# EUROPEAN PATENT APPLICATION

(11) **EP 0 999 205 A1**
(43) Date of publication of application: **10.05.2000**
(21) Application number: 98933913.0
(22) Date of filing: 23.07.1998
(51) Int. Cl.: C07C 237/20, C07C 255/42, C07D 207/09, C07D 213/38, C07D 233/61, C07D 277/28, C07D 295/14, C07D 307/52, C07D 307/79, A61K 31/165, A61K 31/275, A61K 31/34, A61K 31/40, A61K 31/415, A61K 31/425, A61K 31/44, A61K 31/495

(54) **AMINOCYCLOALKANE COMPOUNDS**

(30) Priority: 24.07.1997 JP 19764697
(71) Applicant: Mitsubishi-Tokyo Pharmaceuticals, Inc., Chuo-ku, Tokyo 103-8405 (JP)
(72) Inventor: OHNO, Norio Tokyo Tanabe Company Limited, Tokyo 103-8405 (JP); NAKANO, Masakazu Tokyo Tanabe Company Limited, Tokyo 103-8405 (JP); ENDOH, Jun-Ichi Tokyo Tanabe Company Limited, Tokyo 103-8405 (JP); MIURA, Masataka Tokyo Tanabe Company Limited, Tokyo 103-8405 (JP); AIZAWA, Hideyuki Tokyo Tanabe Company Limited, Tokyo 103-8405 (JP); FUKUZAKI, Athushi Tokyo Tanabe Company Limited, Tokyo 103-8405 (JP); SEIDA, Keiichi Tokyo Tanabe Company Limited, Tokyo 103-8405 (JP)
(74) Representative: Bühling, Gerhard, Dipl.-Chem.
(86) International application number: JP9803299
(87) International publication number: WO9905095

(57) **Abstract**

Aminocycloalkane compounds represented by the following general formula: 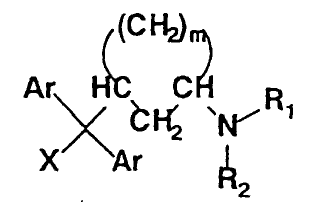 wherein Ar represents a phenyl group or thienyl group with an optional substituent on the ring, X represents a cyano group or carbamoyl group, R₁ and R₂ are each independent, R₁ representing a hydrogen atom or lower alkyl group and R₂ representing a hydrogen atom, lower alkyl group, an -A-B group (where A represents a lower alkylene group that is optionally branched and B represents a phenyl group, pyridyl group, thiazole group, imidazole group, furyl group or pyrrolidinyl group with an optional substituent on the ring) or an alkenyl group, or R₁ and R₂ together with the nitrogen atom to which they are bonded represent 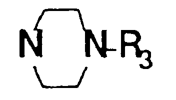 (where R₃ represents a hydrogen atom, a lower alkyl group that is optionally substituted with a phenyl group, or an alkenyl group), and m represents 2, 3 or 4, and pharmacologically acceptable salts thereof.

The compounds have highly selective antagonistic activity for muscarinic M₃ receptor subtypes, and are therefore useful as preventive or therapeutic agents for diseases related to muscarinic M₃ receptor subtypes.

## Description

### Technical Field

The present invention relates to aminocycloalkane compounds, and specifically it relates to aminocycloalkane compounds with selective antagonistic action on muscarinic M₃ receptor subtypes, and to pharmaceutical compositions containing these as effective components.

### Background Art

Muscarinic receptors exist as at least three different subtypes, of which it is reported that M₁ receptor subtypes are distributed in the brain, M₂ receptor subtypes are distributed in the heart and M₃ receptor subtypes are distributed in smooth muscle and glandular tissue.

Drugs with antagonistic action on muscarinic receptors exhibit antispasmodic, analgesic and antisecretory effects, and are therefore useful as therapeutic agents for functional disorders of the intestine, bladder, airways, etc. Drugs with antagonistic action on muscarinic receptors include atropine, scopolamine, oxybutinin, propantheline bromide, ipratropium bromide and the like, which exhibit roughly the same affinity for all three M₁, M₂ and M₃ muscarinic receptor subtypes, and it is known that side-effects are unavoidable due to non-selective competition with acetylcholine for each of these receptors. It has therefore been desirable to provide drugs with highly selective antagonistic action on muscarinic receptors, and especially those which do not produce heart-related side-effects arising from M₂ receptor activity.

As compounds that exhibit selective antagonism for muscarinic M₃ receptor subtypes and exhibit no antihistamine activity, there are known 3-substituted pyrrolidine derivatives (Japanese Laid-Open Patent Publication No. 2-282360, Japanese Patent Public Inspection 4-505927, Japanese Laid-Open Patent Publication No. 7-149640), 3-substituted piperidine derivatives (Japanese Patent Public Inspection 4-500521), carbamate derivatives (WO95/06635), imidazole derivatives (Japanese Laid-Open Patent Publication No. 7-215943), diphenylacetic acid derivatives (Japanese Laid-Open Patent Publication No. 8-291141) and the like, but none of these have exhibited adequate selectivity for muscarinic M₃ receptor subtypes.

On the other hand, U.S. Patent Publication No. 3,772,308 describes cyclopentaneacetamide and cyclopentaneacetonitrile that have anti-inflammatory effects. Both of these cyclopentane compounds differ from the compounds of the present invention in that they have a substituent on the carbon atom adjacent to the cyclopentane ring, and there is no mention of their antagonistic effect on muscarinic receptors.

Also, U.S. Patent Publication No. 2,764,519 describes substituted cyclohexylamines with antispasmodic and parasympathetic blocking effects. However, these also differ from the compounds of the present invention in that the substituents are limited to carbinol groups such as diphenylcarbinol, and there is no reference to selective action on muscarinic M₃ receptor subtypes.

### Disclosure of the Invention

It is an object of the present invention to provide aminocycloalkane compounds with selectivity for muscarinic M₃ receptor subtypes, and especially higher selectivity for muscarinic M₃ receptor subtypes than muscarinic M₂ receptor subtypes, as well as powerful antagonistic action, and to pharmaceutical compositions containing these as effective components.

As a result of much diligent research on compounds with antagonistic action on muscarinic M₃ receptor subtypes, the present inventors have completed the present invention upon finding that aminocycloalkane compounds are represented by general formula (I): 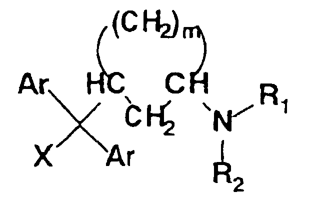 wherein Ar represents a phenyl group or thienyl group with an optional substituent on the ring, X represents a cyano group or carbamoyl group, R₁ and R₂ are each independent, R₁ representing a hydrogen atom or lower alkyl group and R₂ representing a hydrogen atom, lower alkyl group, an -A-B group (where A represents a lower alkylene group that is optionally branched and B represents a phenyl group, pyridyl group, thiazole group, imidazole group, furyl group or pyrrolidinyl group with an optional substituent on the ring) or an alkenyl group, or R₁ and R₂ together with the nitrogen atom to which they are bonded represent 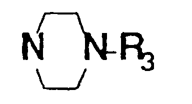 (where R₃ represents a hydrogen atom, a lower alkyl group that is optionally substituted with a phenyl group, or an alkenyl group), and m represents 2, 3 or 4 and their pharmacologically acceptable salts have high selectivity for and powerful antagonistic action on muscarinic M₃ receptor subtypes.

According to the invention, the "optional substituent on Ar" is a monovalent substituent such as a halogen atom, nitro group, amino group, lower alkyl group, lower alkoxy group, mono- or di- lower alkylamino group, lower acyloxy group, lower acylamino group, cyano group, lower alkoxycarbonylalkoxy group or the like, or a divalent substituent such as a methylenedioxy group, trimethylene group, tetramethylene group, ethyleneoxy group or the like, and one or more of the same or different substituents may be present on the ring, with one or two being preferred.

As specific examples of "Ar" there may be mentioned phenyl, thienyl and benzyl, with phenyl being preferred.

The "optional substituent on B" is a monovalent substituent such as a halogen atom, nitro group, amino group, lower alkyl group, lower alkoxy group, mono- or di- lower alkylamino group, lower acyloxy group, lower acylamino group, cyano group, lower alkoxycarbonylalkoxy group or the like, or a divalent substituent such as a methylenedioxy group, trimethylene group, tetramethylene group, ethyleneoxy group or the like, among which halogens and nitro, amino, lower alkyl, lower alkoxy, lower acylamino, cyano, lower alkoxycarbonylalkoxy and ethyleneoxy groups are preferred.

One or more of the same or different substituents among these may be present on the ring, with one or two being preferred.

The "halogen atom" is a fluorine, chlorine or bromine atom, with chlorine being preferred.

The "lower alkyl group" is an alkyl group of 1 to 6 carbon atoms that is optionally branched, and specifically there may be mentioned methyl, ethyl, propyl, isopropyl, butyl, isobutyl, s-butyl, t-butyl, pentyl, isopentyl, neopentyl, t-pentyl and hexyl groups, among which methyl, ethyl, propyl, isopropyl, butyl, isobutyl, s-butyl and t-butyl groups are preferred, and methyl and t-butyl are particularly preferred.

The "lower alkoxy group" is an alkoxy group of 1 to 6 carbon atoms that is optionally branched, of which there may be mentioned methoxy, ethoxy, propoxy, isopropoxy, butoxy, isobutoxy, s-butoxy, t-butoxy, pentyloxy, isopentyloxy, neopentyloxy, t-pentyloxy and hexyloxy groups, among which methoxy, ethoxy, propoxy, isopropoxy, butoxy, isobutoxy, s-butoxy and t-butoxy groups are preferred, and methoxy is particularly preferred.

As "mono- or di- lower alkylamino groups" there may be mentioned methylamino, ethylamino, propylamino, isopropylamino, butylamino, isobutylamino, s-butylamino, t-butylamino, pentylamino, isopentylamino, neopentylamino, t-pentylamino, hexylamino, dimethylamino, diethylamino, dipropylamino, dibutylamino, dipentylamino, dihexylamino, ethylmethylamino, methylpropylamino and butylmethylamino groups, among which methylamino, ethylamino, propylamino, isopropylamino, butylamino, isobutylamino, s-butylamino, t-butylamino, dimethylamino and diethylamino groups are preferred.

The "lower acyloxy group" is an acyloxy group of 1 to 6 carbon atoms, and there may be mentioned formyloxy, acetoxy, propionyloxy, butyroyloxy, isobutyroyloxy, pentanoyloxy and hexanoyloxy groups, among which acetoxy, propionyloxy, butyroyloxy and isobutyroyloxy groups are preferred.

The "lower acylamino group" is an acylamino group of 1 to 6 carbon atoms, and there may be mentioned formylamino, acetamino, propionylamino, butyroylamino, isobutyroylamino, pentanoylamino and hexanoylamino groups, among which acetylamino, propionylamino and butyroylamino groups are preferred, with acetamino being particularly preferred. The "lower alkoxycarbonylalkoxy group" is an alkoxy group of 1 to 3 carbon atoms substituted with an alkoxycarbonyl of 1 to 6 carbon atoms, and specifically there may be mentioned methoxycarbonylmethoxy, ethoxycarbonylmethoxy, propoxycarbonylmethoxy, 2-methoxycarbonylethoxy, 2-ethoxycarbonylethoxy, 2-propoxycarbonylethoxy, 3-methoxycarbonylpropoxy, 3-ethoxycarbonylpropoxy and 3-propoxycarbonylpropoxy groups, among which ethoxycarbonylmethoxy is preferred.

The "lower alkyl group" of R₁, R₂ and R₃ is an alkyl group of 1 to 6 carbon atoms which is optionally branched, and specifically there may be mentioned methyl, ethyl, propyl, isopropyl, butyl, isobutyl, s-butyl, t-butyl, pentyl, isopentyl, neopentyl, t-pentyl and hexyl groups, among which methyl, ethyl, propyl, isopropyl, butyl, isobutyl, s-butyl, t-butyl and 4-methylpentyl groups are preferred, and particularly methyl is preferred as the "lower alkyl group" of R₁, 4-methylpentyl is preferred as the "lower alkyl group" of R₂, and methyl or isopropyl is preferred as the "lower alkyl group" of R₃.

The "lower alkylene group which is optionally branched" is an alkylene group of 1 to 6 carbon atoms, and specifically there may be mentioned methylene, ethylene, trimethylene, tetramethylene, pentamethylene, hexamethylene, methylethylene, ethylethylene, propylene, dimethylethylene and dimethylpropylene groups, among which methylene, ethylene and trimethylene groups are preferred.

The "alkenyl group" is an alkenyl group of 5 to 10 carbon atoms which may be branched, and specifically there may be mentioned 4-methylpenten-3-yl, 3-methylbuten-3-yl, 3-methylbuten-2-yl, 4-methylpenten-4-yl, 8-methylnonen-7-yl, 8-methylnonen-8-yl, 5-methylhexen-4-yl, 5-methylhexen-5-yl, 6-methylhepten-5-yl, 6-methylhepten-6-yl, 7-methylocten-6-yl, 7-methylocten-7-yl, 3-cyclohexylidenepropyl and 2-cyclohexylideneethyl groups, among which 4-methylpenten-3-yl is preferred.

The "pharmacologically acceptable salt" is an inorganic acid salt of hydrochloric acid, nitric acid, sulfuric acid or the like, an organic acid salt or acetic acid, citric acid, fumaric acid, tartaric acid or the like, a sulfonic acid salt of methanesulfonic acid, p-toluenesulfonic acid (hereunder referred to as tosylic acid) or the like, or an amino acid salt of alanine, leucine, glutamic acid, glutamine or the like.

Because compound (I) has at least one asymmetrical carbon in the molecule, it exists as an optical isomer. These optical isomers and their mixtures are all encompassed by the invention.

The compounds of the invention are sometimes isolated as hydrates, solvates or crystalline polymorphic substances, and all of these are also encompassed by the invention.

The compounds of the invention have selective and powerful antagonistic action on muscarinic M₃ receptor subtypes, and can therefore be used as preventive or therapeutic agents for diseases related to muscarinic M₃ receptor subtypes, and particularly digestive tract diseases such as irritable bowel syndrome, spastic colitis and diverticulitis; urologic diseases such as urinary incontinence and frequent urination; respiratory diseases such as chronic obstructive pulmonary disease, asthma, pulmonary fibrosis and rhinitis; and central nervous system conditions such as drug-induced nausea, vomiting, motin sickness, Meniere's disease, etc.

Compound (I) can be produced by any of the following methods. 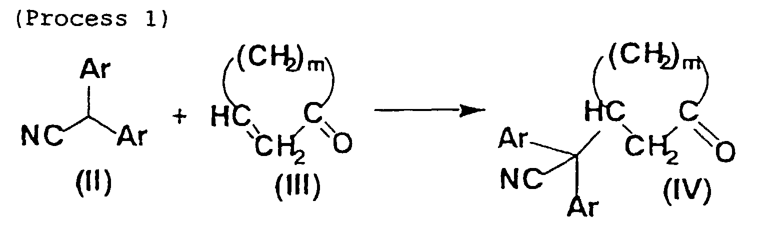 wherein Ar and m are the same as in general formula (I).

Compound (II) and Compound (III) are placed in an organic solvent such as dry tetrahydrofuran (hereunder, "THF"), ether, dioxane, dimethoxyethane, diethyleneglycol dimethyl ether or toluene, and then a basic reagent such as methyllithium, n-butyllithium, s-butyllithium, t-butyllithium, t-butoxypotassium, phenyllithium, sodiumamide, lithium diisopropylamide, triethylamine, diisopropylamine or the like is added at from 0.001 to 2 equivalents, and preferably from 0.01 to 0.1 equivalents with respect to compound (II), from -78°C to 0°C, and preferably from -20°C to 0°C. The mixture is then stirred from 5 minutes to 6 hours and preferably from 10 minutes to one hour, from the same temperature to the boiling point of the solvent and preferably from -20°C to 0°C, and after completion of the reaction a normal purification procedure is used to obtain compound (IV). 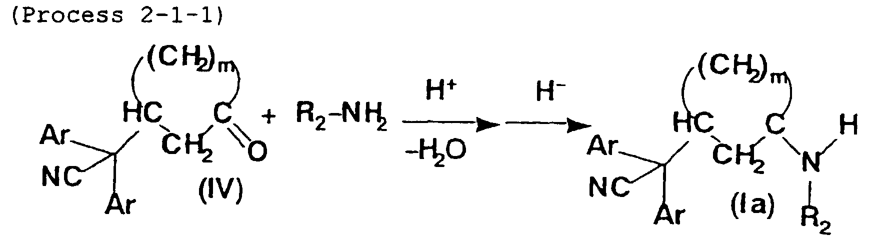 wherein Ar and m are the same as in general formula (I), and R₂ represents a hydrogen atom, lower alkyl group, an -A-B group (where A represents a lower alkylene group that is optionally branched and B represents a phenyl group, pyridyl group, thiazole group, imidazole group, furyl group or pyrrolidinyl group with an optional substituent on the ring) or an alkenyl group.

Compound (IV) and R₂-NH₂ are stirred in a non-water-soluble organic solvent such as benzene, toluene, xylene, mesitylene, chloroform or dichloromethane in the presence of an acid such as benzenesulfonic acid, tosylic acid or boron trifluoride, from room temperature to the boiling point of the solvent, and preferably at the boiling point of the solvent, while removing the water produced. After the residue obtained by concentrating the reaction solution is dissolved in an alcohol such as methanol, ethanol or 2-propanol, it is subjected to a reduction reaction to obtain compound (Ia). The reduction reaction may be a reaction using sodium borohydride, sodium borocyanohydride or the like, or a catalytic reduction reaction using a transition metal such as palladium or Raney nickel as the catalyst.

Compound (Ia) can also be directly obtained by reductive amination of R₂-NH₂ and compound (IV) in an organic solvent such as THF or dichloroethane, using sodium borohydride triacetate, sodium borocyanohydride or the like. 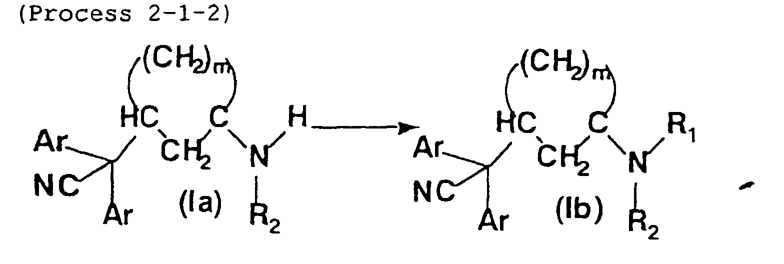 wherein Ar, R₂ and m are the same as in (Process 2-1-1), and R₁ represents a lower alkyl group.

Compound (Ia) and an alkylating agent are reacted in an organic solvent such as acetonitrile, THF, N, N-dimethylformamide or acetone, preferably in the presence of an inorganic base such as sodium carbonate, potassium carbonate, sodium bicarbonate or potassium bicarbonate or an organic base such as triethylamine or N-methylmorpholine, at from room temperature to the boiling point of the solvent, and preferably at the boiling point of the solvent, to obtain compound (Ib).

As the "alkylating agent" there may be mentioned lower alkyl halides, lower alkyl alkylsulfonates, lower alkyl arylsulfonates and the like.

A formalin-formic acid mixture may be also used as a methylating agent. 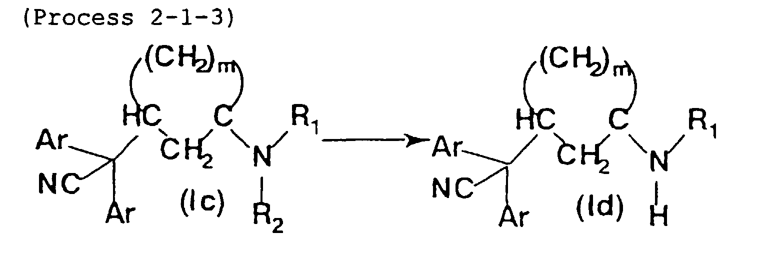 wherein Ar, R₁ and m are the same as in (Process 2-1-2), and R₂ represents a benzyl group.

Compound (Ic) is subjected to a catalytic reduction to obtain reaction to obtain compound (Id). 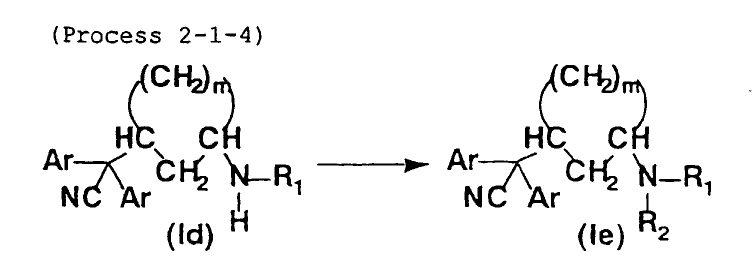 wherein Ar, R₁ and m are the same as in (Process 2-1-3), and R₂ represents a lower alkyl group, an -A-B group (where A represents a lower alkylene group that is optionally branched and B represents a phenyl group, pyridyl group, thiazole group, imidazole group, furyl group or pyrrolidinyl group with an optional substituent on the ring) or an alkenyl group.

Compound (Id) is subjected to alkylating or an alkenylating reaction according to (Process 2-1-2) to obtain compound (Ie). 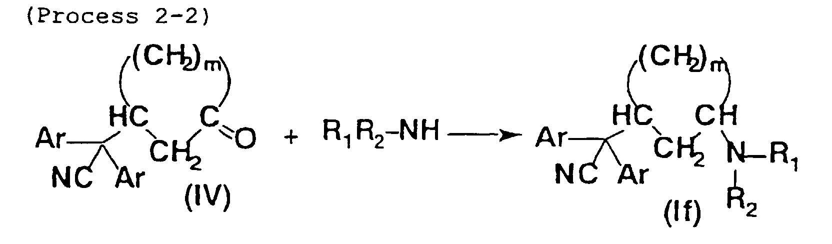 wherein Ar, R₁, R₂ and m are the same as in general formula (I).

Compound (IV) and R₁R₂-NH are subjected to reductive amination in an organic solvent such as THF or dichloroethane, using sodium borohydride triacetate, sodium borocyanohydride or the like, to directly obtain compound (If). 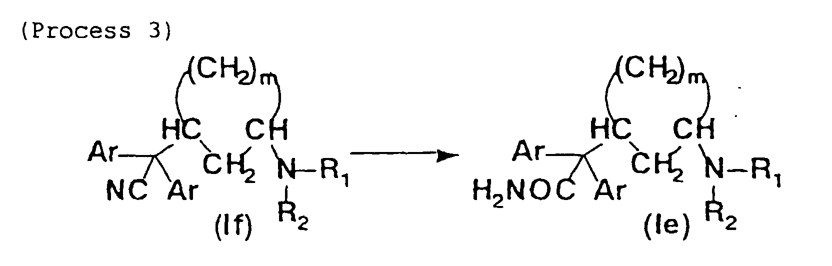 wherein Ar, R₁, R₂ and m are the same as in general formula (I).

The acetonitrile compound (If) is hydrolyzed to obtain the acetamide compound (Ig). The hydrolysis may be carried out by a method of heating under acidic conditions with sulfuric acid or the like, or a method of treatment with hydrogen peroxide under alkali conditions. When R₂ of compound (If) is a benzyl group, it may be obtained by the same method as in (Process 2-1-2) or (Process 2-1-4) after debenzylation reaction by the same method as (Process 2-1-3).

Thus, the compounds of the invention can also be obtained from compounds (Ia) and (Ib) by appropriate combination of the aforementioned processes.

The pharmacologically acceptable salt of compound (I) can be produced by treating compound (I) with an inorganic acid salt of hydrochloric acid, nitric acid, sulfuric acid or the like, an organic acid such as acetic acid, citric acid, fumaric acid, tartaric acid or the like, a sulfonic acid such as methanesulfonic acid, tosylic acid or the like, or an amino acid such as alanine, leucine, glutamic acid, glutamine or the like.

The optical isomers of compound (I) can be optically resolved by allowing an acidic optical resolving agent to act on compound (I) to create a crystalline diastereomer salt, and then recrystallizing. Compound (I) or its modified compounds can also be optically resolved by utilizing an enantiomer-separating technique with high performance liquid chromatography using a chiral stationary phase.

Optically active compounds may also be used in the production processes described above to obtain optically active forms of compound (I).

As forms of administration of the compounds of the invention there may be mentioned oral forms such as tablets, capsules, granules, powders, inhalants and syrups, and parenteral forms such as injections and suppositories.

The compounds of the invention can thus be used as preventive or therapeutic agents for diseases related to muscarinic M₃ receptor subtypes, and particularly digestive tract diseases such as irritable bowel syndrome, spastic colitis and diverticulitis; urologic diseases such as urinary incontinence and frequent urination; respiratory diseases such as chronic obstructive pulmonary disease, asthma, pulmonary fibrosis and rhinitis; and central nervous system conditions such as drug-induced nausea, vomiting, motin sickness, Meniere's disease, etc.

### Best Mode for Carrying Out the Invention

The present invention will now be explained in detail by way of examples and production examples, with the understanding that the invention is in no way limited thereby.

### (Production Example 1)

### 2-(3-Oxocyclopentyl)-2,2-diphenylacetonitrile

Potassium tert-butoxide(0.56 g) was added to a THF (1 liter) solution containing diphenylacetonitrile (98.6 g) and 2-cyclopenten-1-one (41.9 ml) at below -10°C under an argon atmosphere, and the mixture was stirred for 30 minutes. After adding 6 N hydrochloric acid (0.83 ml) to the reaction mixture, the solvent was distilled under reduced pressure. Water was added to the residue, and then extracted with ethyl acetate. Organic solvent was washed with brine, dried over anhydrous sodium sulfate. The solvent was distilled under reduced pressure, isopropyl ether (250 ml) was added to the residue, and then the precipitate was filtered and dried to give the title compound as a white powder (yield: 121.5 g, 88%).
¹H-NMR(CDCl₃) δ:7.51-7.29(10H,m), 3.36(1H,m),2.49-2.16 (4H, m), 2.00 (2H,m).

### (Production Example 2)

### 2-(3-Oxocyclohexyl)-2,2-diphenylacetonitrile

The title compound was obtained from 2-cyclohexen-1-one according to the method of (Production Example 1), as colorless crystals (yield: 14.5 g, quantitative).
¹H-NMR(CDCl₃) δ:7.51-7.28(10H,m), 2.94(1H,m),2.46-2.34 (4H, m),2.12 (1H,m),1.92-1.69(3H,m).

### (Production Example 3)

### 2-(3-Oxocycloheptyl)-2,2-diphenylacetonitrile

The title compound was obtained from 2-cyclohepten-1-one according to the method of (Production Example 1), as colorless crystals (yield: 2.61 g, 57%).
¹H-NMR(CDCl₃) δ:7.51-7.23(10H,m), 2.92-2.70(2H,m),2.57-2.43 (3H,m), 2.04-1.83(3H,m),1.73-1.26(3H,m).

### (Example 1)

### 2-[3-(Benzylamino)cyclopentyl]-2,2-diphenylacetonitrile hydrochloride

A mixture of containing 2-(3-oxocyclopentyl)-2,2-diphenylacetonitrile (27.5 g), benzylamine (10.9 ml) tosyl acid monohydrate (18.9 g) and toluene (500 ml) was stirred for 5 hours in a Dean-Stark apparatus under reflux while removing the water produced during the reaction, then cooled. Reaction mixture was concentrated, then dissolved in ethanol (500 ml). Sodium borohydride (15.1 g) was added to the ethanol solution while cooling on ice bath, and the mixture was stirred at room temperature for one hour. The reaction mixture was then acidified with 1 N hydrochloric acid while cooling on ice, and then concentrated. Water (300 ml) was added to the residue, alkalized with sodium bicarbonate, and then extracted with ethyl acetate (2 x 1 liter). The organic solvent was washed with water (300 ml), brine (2 x 150 ml), dried over anhydrous magnesium sulfate and concentrated. The oily residue was dissolved in ethyl acetate (100 ml), and a 4 N hydrogen chloride /ethyl acetate solution (40 ml) was added. The precipitate was filtered and dried to give the title compound as colorless crystals (yield: 13.1 g, 33%).
¹H-NMR(CDCl₃) δ:10.1(2H,brs), 7.58-7.19(15H,m), 3.98 (2H, brs ), 3.34(1H,m),3.00(1H,m),2.11-1.94(2H,m),1.85-1.68(4H,m).
Melting point: >230°C.

### (Example 2)

### 2-[3-(N-Benzylmethylamino)cyclopentyl]-2,2-diphenylacetonitrile

A mixture of 2-[3-(benzylamino)cyclopentyl]-2,2-diphenylacetonitrile hydrochloride (12.1 g), formic acid (20.0 ml) and a 37% formaldehyde aqueous solution (5.0 ml) was stirred for 11 hours under reflux. After cooling, water (300 ml) was added to the reaction mixture, alkalized with sodium bicarbonate, then extracted with ethyl acetate (2 x 1 liter). The organic solvent was washed with water (300 ml) , brine (2 x 300 ml), dried over anhydrous magnesium sulfate and concentrated. The oily residue was purified by silica gel column chromatography [eluant: hexane containing ethyl acetate (20%)] to give the title compound as colorless crystals (yield: 10.6 g, 93%).
¹H-NMR(CDCl₃) δ:7.49-7.20(15H,m),3.51(1H,d,J=13.4Hz),3.45(1 H,d,J=13.4Hz),3.08(1H,m),2.89(1H,m),2.11(3H,s),1.93-1.65(6 H,m).
Melting point: 103-104°C

### (Example 2-2)

### 2-[3-(N-Benzylmethylamino)cyclopentyl]-2,2-diphenylacetonitrile

Sodium triacetoxy borohydride (100.0 g) was added to a THF (1 liter) solution containing 2-(3-oxocyclopentyl)-2,2-diphenylacetonitrile (82.6 g), N-methylbenzylamine (38.7 ml) and acetic acid (17.2 ml) under an argon atmosphere, and the mixture was stirred for one hour. Water (100 ml) was added to the reaction mixture and after termination of the reaction, a sodium hydroxide aqueous solution (1.5 liters) was added for alkalinity and extraction was performed with ethyl acetate (1.5 liters, 2 x 500 ml). Extracts were washed water (200 ml), brine (200 ml), dried over anhydrous magnesium sulfate and concentrated to give the title compound as light yellow crystals (yield: 113.16 g, 99%).

### (Example 3)

### 2-[3-(N-Benzylmethylamino)cyclopentyl]-2,2-diphenylacetamide)

After adding 70% sulfuric acid (7.0 ml) to an acetic acid (5.0 ml) solution containing 2-[3-(N-benzylmethylamino) cyclopentyl]-2,2-diphenylacetonitrile (2.15 g), the mixture was stirred at 120°C for 12 hours. The reaction mixture was cooled to room temperature and poured onto ice (30 g). The resulting aqueous solution was alkalized with an aqueous 10M sodium hydroxide while cooling on ice, the alkaline aqueous solution was extracted with ethyl acetate (2 x 200 ml). The ethyl acetate layer was washed with water (2 x 40 ml) , brine (2 x 40 ml), dried over anhydrous magnesium sulfate and concentrated. The oily residue was purified by silica gel column chromatography [eluant: hexane containing ethyl acetate (67%)] to give the title compound as an oily substance (yield: 1.22 g, 56%).
¹H-NMR(CDCl₃) δ:7.43-7.18(15H,m),5.41(2H,brs),3.43(1H,m),3. 38(2H,brs), 2.86(1H,m),
   2.05(1H,m),1.98(3H,s),1.85(2H,m),1.34(3H,m).

### (Example 4)

### (A) 2-(3-Methylaminocyclopentyl)-2,2-diphenylacetamide hydrochloride

A mixture of 2-[3-N-benzylmethylamino]cyclopentyl]-2,2-diphenylacetamide (1.18 g), 1 N hydrochloric acid (5 ml), ethanol (100 ml) and 10% Pd/C (0.3 g) was stirred for one hour at room temperature under a hydrogen atmosphere. The mixture was filtered, and the filtrate was concentrated to give the title compound as a colorless powder (yield: 1.02 g, quantitative).
¹H-NMR(CDCl₃) δ:9.63(1H,brs),8.78(1H,brs),7.34-7.17(11H,m), 5.71(1H,brs), 3.53(1H,m), 3.27(1H,m), 2.63(3H,brs), 2.40-2.30(2H,m), 2.12-1.88(4H,m).

### (B) 2-(3-Methylaminocyclopentyl)-2,2-diphenylacetamide

A saturated sodium bicarbonate aqueous solution was added to the 2-(3-methylaminocyclopentyl)-2,2-diphenylacetamide hydrochloride (1.02 g) [from example (A)] and the alkaline mixture was extracted with ethyl acetate (3 x 100 ml). The extract was with brine (2 x 30 ml), dried over anhydrous magnesium sulfate, and concentrated to give the title compound as a colorless oil (yield 0.79 g, 87%).
¹H-NMR(CDCl₃) δ:7.39-7.23(10H,m),5.64(2H,brs),3.35(1H,m),3. 05(1H,m),2.31(3H,s),2.21(2H,m),1.83(2H,m),1.50(1H,m),1.26( 2H,m).

### (Example 5)

### 2-[3-(N-Phenethylmethylamino)cyclopentyl]-2,2-diphenylacetamide

A mixture of 2-(3-methylaminocyclopentyl)-2,2-diphenylacetamide hydrochloride (103 mg), phenethyl tosylate (111 mg), anhydrous potassium carbonate (48 mg) and acetonitrile (3 ml) was refluxed for 4 hours. After cooling the reaction mixture was filtered, and the filtrate was concentrated to give a brown rubbery substance. This residue was purified by silica gel column chromatography [eluant: ethyl acetate containing ethanol (0-10%)] to give an oily substance (80 mg) which was then crystallized from diisopropyl ether to give the title compound as colorless crystals (yield: 19 mg, 15%).
¹H-NMR(CDCl₃) δ:7.40-7.12(15H,m), 5.35(2H,brs), 3.40(1H,m), 2.84(1H,m), 2.71(2H,m), 2.55(2H,m), 2.19(3H,s), 2.07(1H,m), 1.83(2H,m), 1.37-1.11(3H,m).
Melting point: 84-87°C

### (Production Example 4)

### 3-Phenylpropyl tosylate

Tosyl chloride (9.53 g) was added to mixture of triethylamine (6.97 ml) 3-phenyl-1-propanol (6.76 ml) and diethyl ether (100 ml)with ice bath cooling. After removing the ice bath and stirring for 5 hours, the mixture was heated to reflux and stirred for 6 hours. The reaction mixture was filtered and the filtrate was concentrated. The residue was dissolved in ethyl acetate (500 ml), washed with water (3 x 50 ml), brine (2 x 50 ml), dried over anhydrous magnesium sulfate and concentrated. The oily residue was purified by silica gel column chromatography [eluant: hexane containing ethyl acetate (0-10%)] to give the title compound as a colorless oil (yield: 10.5 g, 72%).
¹H-NMR(CDCl₃) δ:7.79(2H,d,J=8.1Hz), 7.34(2H,d,J=8.4Hz), 7.26-7.17(3H,m), 7.07(2H,d,J=6.8Hz), 4.03(2H,t,J=5.9Hz), 2.65(2H,t,J=7.3Hz), 2.46(3H,s), 1.96(2H,m).

### (Example 6)

### 2-[3-[N-(3-Phenylpropyl)methylamino]cyclopentyl]-2,2-diphenylacetamide hydrochloride

A mixture of 2-(3-methylaminocyclopentyl)-2,2-diphenylacetamide hydrochloride (140 mg), 3-phenylpropyl tosylate (177 mg), anhydrous potassium carbonate (70 mg) and acetonitrile (3 ml) was stirred for 3 hours under reflux. The reaction mixture was filtered, and the filtrate was concentrated. The brown rubbery residue was purified by silica gel column chromatography [eluant: ethyl acetate containing methanol (10%)] to give an oily substance. This was then dissolved in ethyl acetate (1 ml), then a 4 N hydrogen chloride/ethyl acetate solution (0.1 ml) was added, and the resulting solid was filtered and dried to give the title compound as a colorless powder (yield: 69 mg, 37%).
¹H-NMR(CDCl₃) δ:9.81(1H,brs),7.31-7.17(15H,m),7.10(1H,brs), 6.78(1H,brs),3.61(1H,m),3.46(1H,m),2.85(2H,m),2.57(2H,m),2. 48(3H,s),2.10(1H,m),1.84(4H,m),1.14(3H,m).

### (Example 7)

### 2-[3-[N-[2-(4-Chlorophenyl)ethyl]methylamino]cyclopentyl]-2,2-diphenylacetamide

A mixture of 2-(3-methylaminocyclopentyl)-2,2-diphenylacetamide hydrochloride (150 mg), 2-(4-chlorophenyl)ethyl tosylate (203 mg) which produced according to the method of (Production Example 4), anhydrous potassium carbonate (105 mg) and acetonitrile (5 ml) was stirred for 8 hours under reflux. The reaction mixture was filtered and the filtrate was concentrated. The oily residue was purified by silica gel column chromatography [eluant: ethyl acetate containing methanol (0-9%)] to give the title compound as a colorless powder (yield: 100 mg, 52%).
¹H-NMR(CDCl₃) δ:7.39-7.20(12H,m), 7.05(2H,d,J=8.6Hz), 5.45(2H,brs),3.38(1H,m),2.84(1H,m),2.67(2H,m),2.49(2H,m), 2.17(3H,s), 2.01(1H,m), 1.81(2H,m), 1.34(1H,m),1.19(2H,m).

### (Example 8)

### 2-[3-[N-[2-(2-Chlorophenyl)ethyl]methylamino]cyclopentyl]-2,2-diphenylacetamide

A mixture of 2-(3-methylaminocyclopentyl)-2,2-diphenylacetamide hydrochloride (150 mg), 2-(2-chlorophenyl)ethyl tosylate (203 mg) which produced according to the method of (Production Example 4), anhydrous potassium carbonate (105 mg) and acetonitrile (5 ml) was stirred for 8 hours under reflux. The reaction mixture was filtered and the filtrate was concentrated. The oily residue was purified by silica gel column chromatography [eluant: ethyl acetate containing methanol (0-9%)] to give the title compound as a colorless oil (yield: 130 mg, 67%).
¹H-NMR(CDCl₃) δ:7.40-7.15(14H,m),5.61(1H,brs),5.46(1H,brs), 3.38(1H,m),2.84(3H,m),2.53(2H,m),2.23(3H,s),2.07(1H,m),1.8 5(2H,m),1.34(1H,m),1.16(2H,m).

### (Example 9)

### 2-[3-[N-[2-(p-Tolyl)ethyl]methylamino]cyclopentyl]-2,2-diphenylacetamide

A mixture of 2-(3-methylaminocyclopentyl)-2,2-diphenylacetamide (220 mg), 2-(4-methylphenyl)ethyl tosylate (210 mg) which produced according to the method of (Production Example 4), anhydrous potassium carbonate (50 mg) and acetonitrile (5 ml) was stirred for 7 hours under reflux. The reaction mixture was filtered and the filtrate was vacuum concentrated. The oily residue was purified by silica gel column chromatography [eluant: ethyl acetate] to give the title compound as a colorless oil (yield: 130 mg, 43%).
¹H-NMR(CDCl₃) δ:7.37-7.26(10H,m), 7.05(4H,m), 5.39(2H,brs), 3.38(1H,m), 2.82(1H,m), 2.67(2H,m),2.51(2H,m), 2.30(3H,s), 2.18(3H,s), 2.15(1H,m), 1.82(2H,m), 1.36-1.11(3H,m).

### (Example 10)

### 2-[3-[N-[2-(4-Methoxyphenyl)ethyl]methylamino]cyclopentyl]-2,2-diphenylacetamide

A mixture of 2-(3-methylaminocyclopentyl)-2,2-diphenylacetamide (230 mg), 2-(4-methoxyphenyl)ethyl tosylate (230 mg) produced according to the method of (Production Example 4), anhydrous potassium carbonate (50 mg) and acetonitrile (5 ml) was stirred for 5 hours under reflux. The reaction mixture was filtered and the filtrate was concentrated. The oily residue was purified by silica gel column chromatography [eluant: ethyl acetate] to give the title compound as a colorless oil (yield: 180 mg, 55%).
¹H-NMR(CDCl₃) δ:7.36-7.27(10H,m), 7.04(2H,d,J=8.1Hz), 6.80(2H,d,J=8.1Hz), 5.75(1H,brs), 5.45(1H,brs), 3.77(3H,s), 3.37(1H,m), 2.81(1H,m), 2.61(2H,m), 2.51(2H,m), 2.17(3H,s), 2.00(1H,m), 1.81(2H,m), 1.36-1.10(3H,m).

### (Example 11)

### 2-[3-[N-(4-Methylpenten-3-yl)methylamino]cyclopentyl]-2,2-diphenylacetamide

A mixture of 2-(3-methylaminocyclopentyl)-2,2-diphenylacetamide (240 mg), 4-methylpenten-3-yl tosylate (200 mg) which produced according to the method of (Production Example 4), anhydrous potassium carbonate (60 mg) and acetonitrile (5 ml) was stirred for 5 hours unfer reflux. The reaction mixture was filtered and the filtrate was concentrated. The oily residue was purified by silica gel column chromatography [eluant: ethyl acetate] to give the title compound as a colorless oil (yield: 140 mg, 47%).
¹H-NMR(CDCl₃) δ:7.40-7.26(10H,m), 5.45(1H,brs),5.38(1H,brs), 5.02(1H,m), 3.38(1H,m), 2.77(1H,m), 2.27(2H,m), 2.11(3H,s), 2.05(3H,m), 1.82(2H,m), 1.67(3H,s), 1.58(3H,s), 1.34-1.07(3H,m).

### (Example 12)

### (A) 2-[3-(N-Benzylmethylamino)cyclopentyl]-2,2-diphenylacetonitrile 2(S)-(-)-2-pyrrolidone-5-carboxylate

(S)-(-)-2-Pyrrolidone-5-carboxylic acid (1.55 g) was added to an acetonitrile (5 ml) solution of 2-[3-(N-benzylmethylamino)cyclopentyl]-2,2-diphenylacetonitrile (4.57 g). The precipitates were collected by suction and recrystallized from acetonitrile to give the title compound as colorless crystals (yield: 2.36 g).
¹H-NMR(CDCl₃) δ:7.47-7.27(15H,m), 6.80(2H,brs), 4.18(2H,m), 4.05(2H,brs), 3.52(1H,m), 3.16(1H,m), 2.49(3H,s), 2.45-2.32(6H,m), 2.22(3H,m), 2.04(3H,m), 1.82(2H,m).
Melting point: 139-144°C.

| Elemental analysis (%) as C₃₇H₄₂N₂O₆·0.25H₂O | | | |
|---|---|---|---|
| Calculated: | C 69.09 | H 6.66 | N 8.71 |
| Found: | C 69.09 | H 6.57 | N 8.65 |

### (B) (-)-2-[3-(N-Benzylmethylamino)cyclopentyl]-2,2-diphenylacetonitrile

Water (50 ml) was added to the 2-[3-(N-benzylmethylamino)cyclopentyl]-2,2-diphenylacetonitrile 2(S)-(-)-2-pyrrolidone-5-carboxylate (2.36 g) which obtained in (A), and the mixture was alkalized with sodium bicarbonate. The reaction mixture was extracted with ethyl acetate (2 x 200 ml), and the organic layer was dried over anhydrous magnesium sulfate and concentrated to give the title compound as colorless crystals (yield: 1.42 g).
[α]_{D}²⁹:-28.2° (c=1.0,MeOH).
¹H-NMR(CDCl₃) δ:7.49-7.20(15H,m), 3.51(1H,d,J=13.4Hz), 3.45(1H,d,J=13.4Hz), 3.08(1H,m), 2.89(1H,m), 2.11(3H,s), 1.95-1.61(6H,m).

### (Example 13)

### (+)-2-[3-(N-benzylmethylamino)cyclopentyl]-2,2-diphenylacetamide

70% Sulfuric acid (7 ml) was added to a acetic acid (2 ml) solution of containing (-)-2-[3-(N-benzylmethylamino) cyclopentyl]-2,2-diphenylacetonitrile (1.19 g), the mixture was stirred at 140°C for 4 hours. After cooling ice (50 g) was added to the reaction solution , then alkalized with an aqueous solution of sodium hydroxide (4.8 g). The aqueous solution was extracted with ethyl acetate (2 x 200 ml), washed with water (40 ml),brine(2 x 40 ml), dried over anhydrous magnesium sulfate. concentrated.The residue was purified by silica gel column chromatography [eluant: ethyl acetate containing hexane (33%)] to give the title compound as a colorless oil (yield: 1.10 g, 88%).
[α]_{D}²⁹:+37.9° (c=1.1,MeOH).
¹H-NMR(CDCl₃) δ:7.43-7.18(15H,m), 5.41(2H,brs), 3.43(1H,m), 3.38(2H,brs), 2.86(1H,m), 2.05(1H,m), 1.98(3H,s), 1.85(2H,m), 1.34(3H,m).

### (Example 14)

### (+)-2-(3-Methylaminocyclopentyl)-2,2-diphenylacetamide hydrochloride

A mixture of (+)-2-[3-(N-benzylmethylamino)cyclopentyl]-2,2-diphenylacetamide (0.98 g), 1 N hydrochloric acid (3 ml), 10% Pd/C (0.3 g) and ethanol (30 ml) was stirred for one hour at room temperature under a hydrogen atmosphere. The reaction mixture was filtered, and the filtrate was concentrated to give the title compound as a colorless amorphous substance (yield: 0.84 g, quantitative).
[α]_{D}²⁵:+9.1° (c=1.0,MeOH).
¹H-NMR(CDCl₃) δ:9.63(1H,brs), 8.78(1H,brs), 7.34-7.17(11H,m), 5.71(1H,brs), 3.53(1H,m), 3.27(1H,m), 2.63(3H,brs), 2.40-2.30(2H,m), 2.12-1.88(4H,m).

### (Example 15)

### (+)-2-[3-(N-Phenethylmethylamino)cyclopentyl]-2,2-diphenylacetamide

A mixture of (+)-2-(3-methylaminocyclopentyl)-2,2-diphenylacetamide hydrochloride (0.28 g), phenethyl tosylate (0.22 g), anhydrous potassium carbonate (0.11 mg) and acetonitrile (3 ml) was stirred for 4 hours under reflux. The reaction mixture was filtered and the filtrate was concentrated. The brown rubbery residue was purified by silica gel column chromatography [eluant: ethyl acetate] to give the title compound as a colorless oil (yield: 0.23 g, 70%).
[α]_{D}²⁹:+33.3° (c=0.95,MeOH).
¹H-NMR(CDCl₃) δ:7.40-7.12(15H,m), 5.35(2H,brs), 3.40(1H,m), 2.84(1H,m), 2.71(2H,m), 2.55(2H,m), 2.19(3H,s), 2.07(1H,m), 1.83(2H,m), 1.37-1.11(3H,m).

### (Example 16)

### 2-(3-Phenethylamino)cyclopentyl-2,2-diphenylacetonitrile

A mixture of 2-(3-oxocyclopentyl)-2,2-diphenylacetonitrile (2.13 g), phenethylamine (0.97 ml) , tosyl acid monohydrate (1.46 g) and toluene (200 ml)was stirred with a Dean-Stark apparatus for 3 hours under reflux while removing the water produced by the reaction. After cooling, the reaction mixture was concentrated. The residue was dissolved in ethanol (200 ml), then sodium borohydride (0.73 g) was added at ice cooling, and the mixture was stirred at room temperature for one hour. After acidifying the reaction mixture with 1 N hydrochloric acid, it was concentrated. The residue was dissolved in water (50 ml) and then alkalized with sodium bicarbonate. The aqueous solution was extracted with chloroform (2 x 200 ml), washed with water (30 ml) , brine(30 ml), dried over anhydrous magnesium sulfate and concentrated. The residue wsa purified by silica gel column chromatography [eluant: chloroform containing methanol (0-1%)] to give the title compound as an oily substance (yield: 0.61 g, 21%).
¹H-NMR(CDCl₃) δ:7.46-7.17(15H,m), 3.16-3.04(2H,m), 2.91-2.80(4H,m), 2.00-1.39(7H,m).

### (Example 17)

### 2-[3-(N-phenethylmethylamino)cyclopentyl]-2,2-diphenylacetonitrile

A mixture of 2-(3-phenethylamino)cyclopentyl-2,2-diphenylacetonitrile (0.61 g), formic acid (1 ml), a 37% formaldehyde aqueous solution (1 ml) and water (5 ml) was stirred for 3 hours under reflux. After cooling the reaction mixture was alkalized by a saturated sodium bicarbonate aqueous solution. The aqueous solution was extracted with ethyl acetate (2 x 80 ml) , washed with brine (2 x 30 ml), dried over anhydrous magnesium sulfate and concentrated. The oily residue was purified by silica gel column chromatography [eluant: ethyl acetate containing hexane (50%)] to give the title compound as an oily substance (yield: 0.54 g, 86%).
¹H-NMR(CDCl₃) δ:7.47-7.15(15H,m), 3.08(1H,m), 2.91(1H,m), 2.78(2H,m), 2.66(2H,m), 2.32(3H,s), 1.90-1.58(6H,m).

### (Example 18)

### 2-[3-(N-Phenethylmethylamino)cyclopentyl]-2,2-diphenylacetamide

2-[3-(N-phenethylmethylamino)cyclopentyl]-2,2-diphenylacetonitrile (100 mg) was dissolved in 70% sulfuric acid (3.5 ml), and the solution was stirred at 140°C for 2 hours. After cooling, reaction mixture was poured onto ice (30 g) and basified with an aqueous 10M sodium hydroxide with cooling. The aqueous solution was extracted with ethyl acetate (2 x 100 ml), washed with water (20 ml), brine (20 ml), dried over anhydrous magnesium sulfate and concentrated. The oily residue was purified by silica gel column chromatography [eluant: chloroform containing methanol (1-9%)] to give an oily substance (20 mg). This was crystallized with diethyl ether to give the title compound as a colorless powder (yield: 3 mg, 3%).
¹H-NMR(CDCl₃) δ:7.39-7.12(15H,m), 5.35(2H,brs), 3.39(1H,m), 2.90(1H,m), 2.71(2H,m), 2.58(2H,m), 2.21(3H,s), 2.05(1H,m), 1.84(2H,m), 1.35-1.17(3H,m).

### (Example 19)

### 2-(3-Phenethylaminocyclohexyl)-2,2-diphenylacetonitrile

The cis form of the title compound was obtained as colorless crystals (yield: 0.69 g, 38%) and the trans form was obtained as a colorless oil (yield: 0.32 g, 18%) from 2-(3-oxocyclohexyl)-2,2-diphenylacetonitrile according to the method of (Example 16).
Cis form: ¹H-NMR(CDCl₃) δ:7.47(4H,m), 7.35-7.16(11H,m), 2.90-2.72(4H,m), 2.56(2H,m), 2.02-1.05(9H,m).
Melting point: 126°C
Transform: ¹H-NMR(CDCl₃) δ:7.44(4H,m), 7.36-7.18(11H,m), 3.00 (2H,m), 2.83 (2H,m), 2.72(2H,m), 1.69-1.25(9H,m).

### (Example 20)

### cis-2-[3-(N-Phenethylmethylamino)cyclohexyl]-2,2-diphenylacetonitrile

The title compound was obtained as a colorless oil from cis-2-(3-phenethylaminocyclohexyl)-2,2-diphenylacetonitrile according to the method of (Example 17) (yield: 0.52 g, 84%).
¹H-NMR(CDCl₃) δ:7.50-7.11(15H,m), 2.72-2.55(6H,m), 2.30(3H,s), 1.85(3H,m), 1.59(1H,m), 1.46-1.23(4H,m).

### (Example 21)

### trans-2-[3-(N-Phenethylmethylamino)cyclohexyl]-2,2-diphenylacetonitrile

The title compound was obtained as a colorless oil from trans-2-(3-phenethylaminocyclohexyl)-2,2-diphenylacetonitrile according to the method of (Example 17) (yield: 0.28 g, 90%).
¹H-NMR(CDCl₃) δ:7.50-7.06(15H,m), 2.97(1H,m), 2.66-2.55(5H,m), 2.01(3H,s),1.81-1.63(3H,m),1.49-1.32(4H,m).

### (Example 22)

### cis-2-[3-(N-Phenethylmethylamino)cyclohexyl]-2,2-diphenylacetamide

The title compound was obtained as colorless crystals from cis-2-[3-(N-phenethylmethylamino)cyclohexyl]-2,2-diphenylacetonitrile according to the method of (Example 18) (yield: 0.19 g, 37%).
¹H-NMR(CDCl₃) δ:7.41-7.14(15H,m),5.47(2H,brs),2.97(1H,m),2. 73-2.63(5H,m),2.27(3H,s),2.03(1H,m),1.80(3H,m),1.44(1H,m), 0.98(1H,m), 0.56(2H,m).

Upon irradiation of the proton at position 1 of the cyclohexane ring, an NOE was measured for the proton at position 3 of the cyclohexane ring.
Melting point: 158-160°C

### (Example 23)

### trans-2-[3-(N-phenethylmethylamino)cyclohexyl]-2,2-diphenylacetamide

The title compound was obtained as a colorless oily product from trans-2-[3-(N-phenethylmethylamino)cyclohexyl]-2,2-diphenylacetonitrile according to the method of (Example 18) (yield: 0.07 g, 52%).
¹H-NMR(CDCl₃) δ:7.41-7.18(15H,m),5.41(1H,brs),5.23(1H,brs), 3.30(1H,m),2.80(4H,m),2.46(1H,m),2.41(3H,s),2.05-1.61(4H,m ),1.41(1H,m),1.07(1H,m),0.85(1H,m),0.67(1H,m).

### (Example 24)

### 2-(3-Benzylaminocyclohexyl)-2,2-diphenylacetonitrile

The title compound was obtained as a colorless oily product from 2-(3-oxocyclohexyl)-2,2-diphenylacetonitrile and benzylamine according to the method of (Example 16) (yield: 2.45 g, 47%).
¹H-NMR(CDCl₃) δ:7.49-7.21(15H,m),3.73(2H,s),2.53(2H,m),2.04 -1.15(9H,m).

### (Example 25)

### 2-[3-(N-Benzylmethylamino)cyclohexyl]-2,2-diphenylacetonitrile

The title compound was obtained as a colorless oily product from 2-(3-benzylaminocyclohexyl)-2,2-diphenylacetonitrile according to the method of (Example 17) (yield: 1.45 g, 61%).
¹H-NMR(CDCl₃) δ:7.50-7.23(15H,m),3.55(1H,d,J=13.0Hz),3.47(1 H,d,J=13.0Hz),2.53(2H,m),2.17(3H,s),1.88(3H,m),1.55(1H,m), 1.47-1.23(4H,m).

### (Example 26)

### 2-[3-(N-Benzylmethylamino)cyclohexyl]-2,2-diphenylacetamide

The title compound was obtained as colorless crystals from 2-[3-(N-benzylmethylamino)cyclohexyl]-2,2-diphenylacetonitrile according to the method of (Example 18) (yield: 0.33 g, 56%)
¹H-NMR(CDCl₃) δ:7.40-7.28(15H,m),5.48(2H,brs),3.53(1H,d,J=1 3.5Hz),3.46(1H,d,J=13.5Hz),2.91(1H,m),2.70(1H,m),2.11(3H,s ),2.04(1H,m),1.80(3H,m),1.42(1H,m),1.06(1H,m),0.73-0.53(2H ,m).
Melting point: 70°C

### (Example 27)

### 2-(3-Methylaminocyclohexyl)-2,2-diphenylacetamide hydrochloride

The title compound was obtained as a colorless amorphous substance from 2-[3-(N-benzylmethylamino)cyclohexyl]-2,2-diphenylacetamide according to the method of (Example 4) (yield: 0.09 g, quantitative).
¹H-NMR(CD₃OD) δ:7.43-7.29(10H,m),3.22(1H,m),3.10(1H,m),2.61 (3H,s),2.26(1H,m),2.09(1H,m),1.87(2H,m),1.56(1H,m),0,96(1H, m),0.63(2H,m).

### (Example 28)

### 2-[3-[N-[2-(4-Methoxyphenyl)ethyl]methylamino]cyclohexyl]- 2,2-diphenylacetamide

The title compound was obtained as a colorless amorphous substance from 2-(3-methylaminocyclohexyl)-2,2-diphenylacetamide hydrochloride according to the method of (Example 10) (yield: 0.18 g, 70%).
¹H-NMR(CDCl₃) δ:7.40-7.26(10H,m),7.06(2H,d,J=8.6Hz),6.80(2H ,d,J=8.6Hz),5.52(2H,brs),3.78(3H,s),2,96(1H,m),2.74-2.55(5 H,m),2.24(3H,s),2.00(1H,m),1.78(3H,m),1.43(1H,m),0.96(1H,m ),0.65-0.49(2H,m).

### (Example 29)

### 2-[3-[N-(3-Phenylpropyl)methylamino]cyclohexyl]-2,2-diphenylacetamide

The title compound was obtained as colorless crystals from 2-(3-methylaminocyclohexyl)-2,2-diphenylacetamide hydrochloride according to the method of (Example 6) (yield: 0.07 g, 53%).
¹H-NMR(CDCl₃) δ:7.39-7.15(15H,m),5.49(1H,brs),5.39(1H,brs), 2.98(1H,m),2.89(1H,m),2.61(2H,m),2.44(2H,m),2.35(3H,s),1.9 8(1H,m),1.78(5H,m),1.42(1H,m),0.96(1H,m),0.66-0.55(2H,m).
Melting point: 119°C

### (Production Example 5)

### 2-(2,3-Dihydrobenzofuran-5-yl)ethyl tosylate

Tosyl chloride (38.9 g) was added to a THF(400 ml) solution of 2-(2,3-dihydrobenzofuran-5-yl) ethanol (33.5 g) and triethylamine (31 ml) at ice bath cooling, and then the mixture was stirred at room temperature. Water and diethyl ether were added to the reaction mixture, then organic solution was washed with brine, dried over anhydrous sodium carbonate, and concentrated. The residue was purified by silica gel column chromatography [eluant: hexane/ethyl acetate = 6/1 to 3/1] to give the title compound (yield: 49.18 g, 76%).
¹H-NMR(CDCl₃) δ:7.69(2H,d,J=8.6Hz),7.28(2H,d,J=8.6Hz),6.94( 1H,s),6.82(1H,dd,J=7.9,1.8Hz),6.65(1H,d,J=7.9Hz),4.54(2H,t d,J=8.6,3.1Hz),4.15(2H,t,J=7.3Hz),3.14(2H,t,J=8.6Hz),2.87( 2H,t,J=7.3Hz),2.44(3H,s).

### (Example 30)

### 2-[3-[N-[2-(2,3-Dihydrobenzofuran-5-yl)ethyl]methylamino] cyclohexyl]-2,2-diphenylacetamide

A mixture of 2-(3-methylaminocyclohexyl)-2,2-diphenylacetamide hydrochloride (90 mg), 2-(2,3-dihydrobenzofuran-5-yl)ethyl tosylate (76 mg), anhydrous potassium carbonate (66 mg) and acetonitrile (4 ml) was stirred for 6 hours wih reflux. The reaction mixture was filtered and then filtrate was concentrated. The oily residue was purified by silica gel column chromatography [eluant: chloroform containing methanol (1-9%)] to give the title compound as colorless crystals (yield: 40 mg, 36%).
¹H-NMR(CDCl₃) δ:7.40-7.30(10H,m),6.98(1H,s),6.87(1H,d,J=7.8 Hz),6.67(1H,d,J=7.8Hz),5.55(2H,brs),4.53(2H,t,J=8.6Hz),3.1 6(2H,t,J=8.6Hz),2.96(1H,m),2.79-2.59(5H,m),2.26(3H,s),2.02 (1H,m),1.79(3H,m),1.44(1H,m),0.97(1H,m),0.58(2H,m).
Melting point: 77°C

### (Example 31)

### 2-(3-Phenethylaminocycloheptyl)-2,2-diphenylacetonitrile

The title compound was obtained as a colorless oily product from (3-oxocycloheptyl)diphenylacetonitrile according to the method of (Example 16) (yield: 0.20 g, 6%).
¹H-NMR(CDCl₃) δ:7.61-7.11(15H,m),2.91(1H,m),2.71-2.43(3H,m) ,1.96-1.36(13H,m).

### (Example 32)

### 2-[3-(N-Phenethylmethylamino)cycloheptyl]-2,2-diphenylacetonitrile

The title compound was obtained as a colorless oil from 2-(3-phenethylaminocycloheptyl)-2,2-diphenylacetonitrile according to the method of (Example 17) (yield: 0.12 g, 57%).
¹H-NMR(CDCl₃) δ:7.51-7.09(15H,m),2.89(1H,m),2.79(1H,m),2.64 (2H,m),2.43(2H,m),2.13(3H,s),1.82(5H,m),1.51-1.34(5H,m).

### (Example 33)

### 2-[3-(N-Phenethylmethylamino)cycloheptyl]-2,2-diphenylacetamide

The title compound was obtained as colorless crystals from 2-[3-(N-phenethylmethylamino)cycloheptyl]-2,2-diphenylacetonitrile according to the method of (Example 18) (yield: 0.05 g, 42%).
¹H-NMR(CDCl₃) δ:7.42-7.14(15H,m),5.31(2H,brs),3.12(1H,s),2. 71-2.55(5H,m),2.28(3H,s),1.62-1.44(3H,m),1.15(1H,m),0.77(1 H,m).
Melting point: 63°C

### (Example 34)

### (A) 2-[3-(N-Benzylmethylamino)cyclopentyl]-2,2-diphenylacetonitrile 2(R)-(+)-2-pyrrolidone-5-carboxylate

The title compound was obtained as colorless crystals from (R)-(+)-2-pyrrolidone-5-carboxylic acid according to the method of (Example 12)(A) (yield: 2.13 g).
¹H-NMR(CDCl₃) δ:7.47-7.28(15H,m),6.76(2H,brs),4.18(2H,m),4. 04(2H,brs),3.53(1H,m),3.14(1H,m),2.48(3H,s),2.46-2.33(6H,m ),2.22(3H,m),2.04(3H,m),1.82(2H,m).
Melting point: 139-144°C

| Elemental analysis (%) as C₃₇H₄₂N₂O₆·0.25H₂O | | | |
|---|---|---|---|
| Calculated: | C 69.09 | H 6.66 | N 8.71 |
| Found: | C 69.02 | H 6.54 | N 8.65 |

### (B) (+)-2-[3-(N-Benzylmethylamino)cyclopentyl]-2,2-diphenylacetonitrile

The title compound was obtained as colorless crystals from the 2-[3-(N-benzylmethylamino)cyclopentyl]-2,2-diphenylacetonitrile 2(R)-(+)-2-pyrrolidone-5-carboxylate of step (A) according to the method of (Example 12)(B) (yield: 1.16 g).
[α]_{D}²⁸:+27.5° (c=1.0,MeOH).
¹H-NMR(CDCl₃) δ:7.48-7.22(15H,m),3.51(1H,d,J=13.5Hz),3.45(1 H,d,J=13.5Hz),3.09(1H,m),2.89(1H,m),2.10(3H,s),1.93-1.60(6 H,m).

### (Example 35)

### (-)-2-[3-(N-Benzylmethylamino)cyclopentyl]-2,2-diphenylacetamide

The title compound was obtained as a colorless oily product from (+)-2-[3-(N-benzylmethylamino)cyclopentyl]-2,2-diphenylacetonitrile according to the method of (Example 13) (yield: 0.58 g, 53%).
[α]_{D}²⁶:-34.4° (c=0.81,MeOH).
¹H-NMR(CDCl₃) δ:7.42-7.18(15H,m),5.41(2H,brs),3.43(1H,m),3. 38(2H,brs),2.84(1H,m),2.05(1H,m),1.98(3H,s),1.85(2H,m),1.3 4(3H,m).

### (Example 36)

### (-)-2-(3-Methylaminocyclopentyl)-2,2-diphenylacetamide hydrochloride

The title compound was obtained as a colorless amorphous substance from (-)-2-[3-(N-benzylmethylamino)cyclopentyl]-2,2-diphenylacetamide according to the method of (Example 14) (yield: 0.50 g, quantitative).
[α]_{D}²⁷:-8.7° (c=1.5,MeOH).
¹H-NMR(CDCl₃) δ:9.79(1H,brs),8.61(1H,brs),7.61(1H,brs),7.33 -7.14(10H,m),5.72(1H,brs),3.54(1H,m),3.26(1H,m),2.64(3H,br s),2.47-2.36(2H,m),2.14-2.00(2H,m),1.89(2H,m).

### (Example 37)

### (-)-2-[3-(N-Phenethylmethylamino)cyclopentyl]-2,2-diphenylacetamide

The title compound was obtained as a colorless oily product from (-)-2-(3-methylaminocyclopentyl)-2,2-diphenylacetamide hydrochloride according to the method of (Example 15) (yield: 0.15 g, 67%).
[α]_{D}²⁸:-32.1° (c=1.5,MeOH).
¹H-NMR(CDCl₃) δ:7.40-7.12(15H,m),5.44(2H,brs),3.40(1H,m),2. 83(1H,m),2.71(2H,m),2.55(2H,m),2.19(3H,s),2.07(1H,m),1.83( 2H,m),1.36-1.11(3H,m).

### (Example 38)

### (-)-2-[3-[N-[2-(4-Nitrophenyl)ethyl]methylamino]cyclopentyl]-2,2-diphenylacetamide

(-)-2-(3-Methylaminocyclopentyl)-2,2-diphenylacetamide hydrochloride and 2-(4-nitrophenyl)ethyl tosylate were used for production according to the method of (Example 15) to give the title compound as a colorless oil (yield: 87 mg, 48%).
MSm/z:458(C₂₈H₃₁N₃O₃+H).
[α]_{D}²⁶:-25.9° (c=0.87,CHCl₃).
¹H-NMR(CDCl₃) δ:8.11(2H,d,J=8.5Hz),7.35-7.26(12H,m),5.43(2H ,brs),3.39(1H,m),2.86(1H,m),2.78(2H,m),2.55(2H,m),2.18(3H, s),2.02(1H,m),1.89-1.71(2H,m),1.37(1H,m),1.13(2H,m).

### (Example 39)

### (-)-2-[3-[N-[2-(4-t-Butylphenyl)ethyl]methylamino]cyclopentyl]-2,2-diphenylacetamide

(-)-2-(3-Methylaminocyclopentyl)-2,2-diphenylacetamide hydrochloride and 2-(4-t-butylphenyl)ethyl tosylate were used for production according to the method of (Example 38) to give the title compound as a colorless oil (yield: 0.14 g, 75%).
[α]_{D}²⁷:-34.3° (c=1.31,MeOH).
¹H-NMR(CDCl₃) δ:7.39-7.22(12H,m),7.07(2H,d,J=7.9Hz),5.72(1H ,brs),5.47(1H,brs),3.38(1H,m),2.83(1H,m),2.67(2H,m),2.54(2 H,m),2.18(3H,s),2.02(1H,m),1.80(2H,m),1.36-1.10(12H,m).

### (Example 40)

### (-)-2-[3-[N-[2-(3,4-Dimethylphenyl)ethyl]methylamino] cyclopentyl]-2,2-diphenylacetamide

(-)-2-(3-Methylaminocyclopentyl)-2,2-diphenylacetamide hydrochloride and 2-(3,4-dimethylphenyl)ethyl tosylate were used for production according to the method of (Example 38) to give the title compound as a colorless oil (yield: 0.12 g, 68%).
MSm/z:441(C₃₀H₃₆N₂O+H).
[α]_{D}²⁸:-35.4° (c=0.95,MeOH).
¹H-NMR(CDCl₃) δ:7.40-7.23(10H,m),7.02(1H,d,J=7.9Hz),6.90(1H ,s),6.87(1H,d,J=7.9Hz),5.46(2H,brs),3.38(1H,m),2.82(1H,m), 2.61(2H,m),2.52(2H,m),2.22(3H,s),2.21(3H,s),2.18(3H,s),2.0 4(1H,m),1.84(2H,m),1.36-1.11(3H,m).

### (Example 41)

### (-)-2-[3-[N-[2-(3-Chlorophenyl)ethyl]methylamino]cyclopentyl]-2,2-diphenylacetamide

(-)-2-(3-Methylaminocyclopentyl)-2,2-diphenylacetamide hydrochloride and 2-(3-chlorophenyl)ethyl tosylate were used for production according to the method of (Example 38) to give the title compound as a colorless oil (yield: 0.13 g, 73%).
MSm/z:447(C₂₈H₃₁N₂OCl+H).
[α]_{D}²⁷:-31.3° (c=1.19,MeOH).
¹H-NMR(CDCl₃) δ:7.40-7.26(10H,m),7.21-7.11(3H,m),7.00(1H,d, J=6.1Hz),5.43(2H,brs),3.38(1H,m),2.82(1H,m),2.65(2H,m),2.5 1(2H,m),2.17(3H,s),2.03(1H,m),1.82(2H,m),1.34-1.11(3H,m).

### (Example 42)

### (-)-2-[3-[N-[2-(4-Ethoxycarbonylmethoxyphenyl)ethyl]methylamino] cyclopentyl]-2,2-diphenylacetamide

(-)-2-(3-Methylaminocyclopentyl)-2,2-diphenylacetamide hydrochloride and 2-(4-ethoxycarbonylmethoxyphenyl)ethyl tosylate were used for production according to the method of (Example 38) to give the title compound as a colorless oil (yield: 0.16 g, 71%).
[α]_{D}²⁸:-31.7° (c=1.40,MeOH).
¹H-NMR(CDCl₃) δ:7.39-7.26(10H,m),7.05(2H,d,J=8.5Hz),6.80(2H ,d,J=8.5Hz),5.41(2H,brs),4.58(2H,s),4.27(2H,dd,J=7.3Hz,14. 0Hz),3.39(1H,m),2.82(1H,m),2.63(2H,m),2.50(2H,m),2.17(3H,s ),2.03(1H,m),1.82(2H,m),1.36-1.10(6H,m).

### (Example 43)

### (-)-2-[3-[N-[2-(4-aminophenyl)ethyl]methylamino]cyclopentyl]-2,2-diphenylacetamide

A mixture of (-)-2-[3-[N-[2-(4-nitrophenyl)ethyl] methylamino]cyclopentyl]-2,2-diphenylacetamide (100 mg), 1 N hydrochloric acid (1 ml), ethanol (10 ml) and 10% Pd/C (20 mg) was stirred at room temperature under a hydrogen atmosphere for one hour. The mixture was filtered, and then filtrate was concentrated to give colorless crystals (yield: 120 mg, quantitative). The crystals (50 mg) were dissolubled in ethyl acetate (100 ml), washed with a aqueous sodium hydroxide (10 ml) , brine (2 x 10 ml), dried over anhydrous magnesium sulfate and concentrated to give the title compound as a colorless oil (yield: 45 mg).
MSm/z:428(C₃₀H₃₃N₃O+H).
[α]_{D}²⁸:-27.6° (c=0.45,CHCl₃).
¹H-NMR(CDCl₃) δ:7.40-7.23(10H,m),6.92(2H,d,J=8.0Hz),6.60(2H ,d,J=8.0Hz),5.44(2H,brs),3.54(2H,brs),3.38(1H,m),2.84(1H,m ),2.58(2H,m),2.50(2H,m),2.18(3H,s),1.95(1H,m),1.82(2H,m),1. 34-1.12(3H,m).

### (Example 44)

### (-)-2-[3-[N-[2-(4-Acetaminophenyl)ethyl]methylamino] cyclopentyl]-2,2-diphenylacetamide

A mixture of (-)-2-[3-[N-[2-(4-aminophenyl)ethyl]methylamino] cyclopentyl]-2,2-diphenylacetamide (27 mg), pyridine (1 ml) and acetic anhydride (1 mg) was stirred for 8 hours. The reaction mixture was vacuum concentrated, and the residue was dissolved in ethyl acetate (80 ml) . The organic solution was washed with a saturated sodium bicarbonate aqueous solution (20 ml) , brine (20 ml), dried over anhydrous magnesium sulfate and concentrated. The oily residue was purified by silica gel column chromatography [eluant: chloroform/methanol/30% ammonia water = 15/1/0.01] to give the title compound as a colorless oil (yield: 13 mg, 59%).
MSm/z:470(C₃₀H₃₅N₃O₂+H).
[α]_{D}²⁶:-30.9° (c=0.13,CHCl₃).
¹H-NMR(CDCl₃) δ:7.39-7.26(12H,m), 7.09-7.06(3H,m), 5.40(2H,brs), 3.38(1H,m), 3.85(1H,m), 2.67(2H,m), 2.52(2H,m), 2.18(3H,s),2.16(3H,s),2.03(1H,m),1.90-1.74(2H,m),1.42-1.08(3H,m).

### (Example 45)

### (-)-2-[3-[N-[2-(2,3-Dihydrobenzofuran-5-yl)ethyl]methylamino] cyclopentyl]-2,2-diphenylacetamide

(-)-2-(3-methylaminocyclopentyl)-2,2-diphenylacetamide hydrochloride and 2-(2,3-dihydrobenzofuran-5-yl)ethyl tosylate were used for production according to the method of (Example 38) to give the title compound as a colorless oil (yield: 0.12 g, 66%).
MSm/z:455(C₃₀H₃₄N₂O₂+H).
[α]_{D}²⁶:-25.8° (c=1.18,CHCl₃).
¹H-NMR(CDCl₃) δ:7.39-7.23(10H,m), 6.97(1H,s),
   6.86(1H,d,J=7.9
   Hz), 6.67(1H,d,J=7.9Hz), 5.44(2H,brs), 4.53(2H,t,J=8.5Hz), 3.39(1H,m), 3.16(2H,t,J=8.5Hz), 2.85(1H,m), 2.62(2H,m), 2.51(2H,m), 2.19(3H,s), 2.04(1H,m), 1.83(2H,m), 1.34-1.13(3H,m).

### (Example 46)

### 2-[3-[N-(4-Methylpentyl)methylamino]cyclopentyl]-2,2-diphenylacetamide hydrochloride

2-[3-[N-(4-Methylpenten-3-yl)methylamino]cyclopentyl]-2,2-diphenylacetamide (140 mg) was dissolved in diisopropyl ether (2 ml), then 4 N hydrogen chloride /ethyl acetate solution (0.2 ml) was added thereto , and then the precipitate was filtered to give colorless crystals (151 mg). A mixture of these crystals (30 mg), ethanol (2 ml) and 10% Pd/C (20 mg) was stirred at room temperature under a hydrogen atmosphere for 5 hours. The reaction mixture was filtered, and the filtrate was concentrated to give the title compound as colorless crystals (yield: 28 mg, 93%)
¹H-NMR(CDCl₃) δ:12.0(1H,brs),7.37-7.27(10H,m),5.53(1H,brs), 5.45(1H,brs),3.59(1H,m),3.42(1H,m),2.83(1H,m),2.54-2.42(4H ,m),2.07-1.49(10H,m),1.14(1H,m),0.87(6H,d,J=6.8Hz).
Melting point: 75.9-77.6°C

### (Example 47)

### (-)-2-[3-[N-[2-(6-Methylpyridin-2-yl)ethyl]methylamino] cyclopentyl]-2,2-diphenylacetamide

Following the method of (Example 38), (-)-2-(3-methylaminocyclopentyl)-2,2-diphenylacetamide hydrochloride (0.15 g), 2-(6-methylpyridyl)ethyl tosylate (0.15 g) which obtained by the same method as Production Example 5 and anhydrous potassium carbonate (0.14 g) were stirred in acetonitrile (5 ml) for 8 hours under reflux. After cooling, the mixture was filtered, and the filtrate was distilled off under reduced pressure. The residue was purified by silica gel column chromatography [eluant: ethyl acetate containing methanol (0-20%)] to give the title compound as a colorless oil (yield: 0.14 g, 67%).
[α]_{D}³¹:-36.0° (c=1.29,CHCl₃).
¹H-NMR(CDCl₃) δ:7.47-7.22(11H,m),6.92(2H,m),5.65(1H,brs),5. 47(1H,brs),3.37(1H,m),2.89-2.83(3H,m),2.72-2.66(2H,m),2.50 (3H,s),2.19(3H,s),2.24(1H,m),1.79(2H,m),1.38-1.10(3H,m).
MSm/z:428(C₂₈H₃₃N₃O+H).

### (Example 48)

### (-)-2-[3-[N-[2-(Pyridin-2-yl)ethyl]methylamino]cyclopentyl]-2,2-diphenylacetamide

(-)-2-(3-Methylaminocyclopentyl)-2,2-diphenylacetamide (0.20 g) and 2-(2-pyridyl)ethyl tosylate (0.18 g) were used for production according to the method of (Example 38) to give the title compound as a colorless oil (yield: 0.20 g, 75%).
[α]_{D}²⁸:-21.9° (c=2.02,CHCl₃).
¹H-NMR(CDCl₃) δ:8.48(1H,d,J=4.9Hz),7.55(1H,dd,J=7.3Hz,7.9Hz ),7.38-7.24(10H,m),7.09(2H,m),5.86(1H,brs),5.48(1H,brs),3. 36(1H,m),2.89(3H,m),2.70(2H,m),2.19(3H,s),2.04(1H,m),1.82( 2H,m),1.37-1.08(3H,m).
MSm/z:414(C₂₇H₃₁N₃O+H).

### (Example 49)

### (-)-2-[3-[N-[trans-3-(6-Methylpyridin-2-yl)propen-2-yl]methylamino]cyclopentyl]-2,2-diphenylacetamide

(-)-2-(3-methylaminocyclopentyl)-2,2-diphenylacetamide hydrochloride (0.21 g) and trans-3-(2-(6-methylpyridyl)) acrylaldehyde (0.09 g) were used for production according to the method of (Example 2-2) to give the title compound as a yellow oil (yield: 0.10 g, 88%).
[α]_{D}³¹:-38.5° (c=0.99,CHCl₃).
¹H-NMR(CDCl₃) δ:7.48(1H,t,J=7.3Hz),7.40-7.25(10H,m),7.13(1H ,d,J=7.9Hz),6.96(1H,d,J=7.3Hz),6.57(2H,m),5.70(1H,brs),5.4 7(1H,brs),3.38(1H,m),3.13(2H,d,J=5.5Hz),2.84(1H,m),2.51(3H, s),2.12(3H,s),2.07(1H,m),1.82(2H,m),1.39-1.15(3H,m).
MSm/z:440(C₂₉H₃₃N₃O+H).

### (Example 50)

### (-)-2-[3-[N-[3-(6-Methylpyridin-2-yl)propyl]methylamino] cyclopentyl]-2,2-diphenylacetamide

A mixture of the (-)-2-[3-[N-[trans-3-(6-methylpyridin-2-yl)propen-2-yl]methylamino]cyclopentyl]-2,2-diphenylacetamide of Example 49 (0.13 g), ethanol (5 ml) and 10% Pd-C (0.04 g) was stirred at room temperature under a hydrogen atmosphere for one hour. The mixture was filtered, and the filtrate was concentrated. The oily residue was purified by silica gel column chromatography [eluant: chloroform/methanol = 30/1] to give the title compound as a colorless oil (yield: 0.04 g, 31%).
[α]_{D}³⁰:-7.8° (c=0.49,CHCl₃).
¹H-NMR(CDCl₃) δ:7.47(1H,t,J=7.3Hz),7.36-7.28(10H,m),6.97(1H ,d,J=7.3Hz),6.96(1H,d,J=7.3Hz),5.81(1H,brs),5.51(1H,brs),3. 44(2H,m),2.82-2.71(4H,m),2.48(3H,s),2.42(3H,s),2.20-2.11( 3H,m),1.99-1.89(2H,m),1.67-1.50(3H,m).
MSm/z:442(C₂₉H₃₅N₃O+H).

### (Example 51)

### (-)-2-[3-[N-[2-(2-Methylimidazol-1-yl)ethyl]methylamino] cyclopentyl]-2,2-diphenylacetamide

(-)-2-(3-Methylaminocyclopentyl)-2,2-diphenylacetamide hydrochloride (0.14 g: 0.4 mmol) and 2-(2-methylimidazol-1-yl)ethyl tosylate (0.12 g: 0.44 mmol) which obtained by the same method as Production Example 5 were used for production according to the method of (Example 38) to give the title compound as a crystalline powder from (yield: 0.03 g, 15%).
Melting point: 127-128.5°C
[α]_{D}²⁹:-17.0° (c=0.19,CHCl₃).
¹H-NMR(CDCl₃) δ:7.12-7.52(10H,m),6.84(1H,d,J=1.22Hz),6.75(1 H,s),5.29-5.62(2H,brs),3.79(2H,dd,J=7.32,6.71Hz),3.24-3.44 (1H,m),2.70-2.90(1H,m),2.51(1H,dd,J=7.32,6.71Hz),2.30(3H,s ),2.13(3H,s),1.56-2.04(3H,m),1.22-1.48(1H,m),0.92-1.20(2H, m).
MSm/z:417(C₂₆H₃₂N₄O+H).

### (Example 52)

### (-)-2-[3-[N-[(6-Methylpyridin-2-yl)methyl]methylamino] cyclopentyl]-2,2-diphenylacetamide

(-)-2-(3-Methylaminocyclopentyl)-2,2-diphenylacetamide hydrochloride (0.19 g), 6-methylpyridine-2-aldehyde (0.067 g), sodium borohydride triacetate (0.19 g) and acetic acid (0.03 ml) were stirred for one hour in tetrahydrofuran (4 ml) at room temperature under an argon atmosphere. Water (1 ml) was added, then stirred for 10 minutes. Water and a sodium hydroxide aqueous solution were added until alkalinity, and extracted with ethyl acetate. Organic solution was washed with water, dried over anhydrous sodium sulfate and concentrated. Oily residue was purified by silica gel column chromatography [eluant: ethyl acetate/methanol = 9/1] to give the title compound as a colorless amorphous substance (yield: 0.14 g, 63%).
[α]_{D}³⁰:-21.8° (c=1.39,CHCl₃).
¹H-NMR(CDCl₃) δ:7.48(1H,t,J=7.3Hz),7.40-7.26(10H,m),7.14(1H ,d,J=7.3Hz),6.97(1H,d,J=7.3Hz),5.47(2H,brs),3.55(1H,d,J=14. 0Hz),3.49(1H,d,J=14.0Hz),3.37(1H,m),2.90(1H,m),2.51(3H,s), 2.08(1H,m),2.05(3H,m),1.93-1.79(2H,m),1.41-1.20(3H,m).
MSm/z:414(C₂₇H₃₁N₃O+H).

### (Example 53)

### (+)-2-[3-[N-[(6-Methylpyridin-2-yl)methyl]methylamino] cyclopentyl]-2,2-diphenylacetamide

(+)-2-(3-Methylaminocyclopentyl)-2,2-diphenylacetamide hydrochloride (0.19 g) and 6-methylpyridine-2-aldehyde (0.07 g) were used for production according to the method of (Example 52) to give the title compound as a colorless oil (yield: 0.11 g, 51%).
[α]_{D}²⁷:+23.1° (c=1.12,CHCl₃).
¹H-NMR(CDCl₃) δ:7.48(1H,t,J=7.3Hz),7.40-7.23(10H,m),7.14(1H ,d,J=7.3Hz),6.96(1H,d,J=7.3Hz),5.71(1H,brs),5.46(1H,brs),3. 54(1H,d,J=14.0Hz),3.48(1H,d,J=14.0Hz),3.37(1H,m),2.92(1H,m ),2.50(3H,s),2.08(1H,m),2.05(3H,m),1.90-1.78(2H,m),1.41-1.23(3H,m).
MSm/z:414(C₂₇H₃₁N₃O+H).

### (Example 54)

### (-)-2-[3-[N-(3-Methoxybenzyl)methylamino]cyclopentyl]-2,2-diphenylacetamide

(-)-2-(3-Methylaminocyclopentyl)-2,2-diphenylacetamide hydrochloride (0.14 g: 0.4 mmol) and meta-anisaldehyde (0.05 g: 0.4 mmol) were used for production according to the method of (Example 52) to give the title compound as an oily substance (yield: 0.05 g, 29%).
[α]_{D}³²:-26.0° (c=0.35,CHCl₃).
¹H-NMR(CDCl₃) δ:7.12-7.46(11H,m),6.68-6.86(3H,m),5.47(2H,br s),3.78(3H,s),3.22-3.48(3H,m),2.66-2.92(1H,m),1.68-2.16(6H ,m),1.12-1.50(3H,m).
MSm/z:429(C₂₈H₃₂N₂O₂+H).

### (Example 55)

### (-)-2-[3-[N-(3-Methylbenzyl)methylamino]cyclopentyl]-2,2-diphenylacetamide

(-)-2-(3-Methylaminocyclopentyl)-2,2-diphenylacetamide hydrochloride (0.14 g: 0.4 mmol) and meta-tolualdehyde (0.05 g: 0.4 mmol) were used for production according to the method of (Example 52) to give the title compound as an oily substance (yield: 0.04 g, 23%).
[α]_{D}²⁷:-27.6° (c=0.38,CHCl₃).
¹H-NMR(CDCl₃) δ:7.22-7.48(10H,m),7.12-7.18(1H,m),6.90-7.08( 3H,m),5.29-5.64(2H,brs),3.02-3.48(3H,m),2.72-2.94(1H,m),2. 31(3H,s),1.68-2.14(6H,m),1.06-1.48(3H,m).
MSm/z:413(C₂₈H₃₂N₂O+H).

### (Example 56)

### (-)-2-[3-[N-(2-Picolyl)methylamino]cyclopentyl]-2,2-diphenylacetamide

(-)-2-(3-methylaminocyclopentyl)-2,2-diphenylacetamide hydrochloride (0.17 g: 0.5 mmol) and pyridine-2-aldehyde (0.05 g: 0.5 mmol) were used for production according to the method of (Example 52) to give the title compound as a colorless amorphous substance (yield: 0.05 g, 26%).
[α]_{D}²⁶:-21.4° (c=1.03,CHCl₃).
¹H-NMR(CDCl₃) δ:8.49(1H,d,J=4.22Hz),7.56-7.62(1H,m),7.23-7.40(11H,m),7.09-7.13(1H,m),5.48(2H,broad),3.51-3.60(2H,m) ,3.33-3.49(1H,m),2.86-2.98(1H,m),2.09-2.19(1H,m),2.04(3H,s ),1.80-1.99(2H,m),1.25-1.47(3H,m).
MSm/z:400(C₂₅H₂₉N₃O+H).

### (Example 57)

### (-)-2-[3-[N-(3-Picolyl)methylamino]cyclopentyl]-2,2-diphenylacetamide

(-)-2-(3-Methylaminocyclopentyl)-2,2-diphenylacetamide hydrochloride (0.21 g) and pyridine-3-aldehyde (0.06 g) were used for production according to the method of (Example 52) to give the title compound as a colorless oil (yield: 0.10 g, 42%).
[α]_{D}³⁰:-24.7° (c=1.12,CHCl₃).
¹H-NMR(CDCl₃) δ:8.44(2H,m),7.56(1H,d,J=7.3Hz),7.42-7.27(10H ,m),7.20(1H,m),5.51(2H,brs),3.37(3H,m),2.87(1H,m),2.09-1.8 1(3H,m),1.97(3H,s),1.40(1H,m),1.33-1.20(2H,m).
MSm/z:400(C₂₆H₂₉N₃O+H).

### (Example 58)

### (-)-2-[3-[N-(4-Picolyl)methylamino]cyclopentyl]-2,2-diphenylacetamide

(-)-2-(3-Methylaminocyclopentyl)-2,2-diphenylacetamide hydrochloride (0.21 g) and pyridine-4-aldehyde (0.06 g) were used for production according to the method of (Example 52) to give the title compound as a colorless oil (yield: 0.16 g, 67%).
[α]_{D}³⁰:-21.3° (c=1.20,CHCl₃).
¹H-NMR(CDCl₃) δ:8.46(2H,d,J=5.8Hz),7.41-7.27(10H,m),7.16(2H ,d,J=5.8Hz),5.46(2H,brs),3.40(1H,m),3.36(2H,s),2.87(1H,m), 2.05-1.74(3H,m),1.98(3H,s),1.40(1H,m),1.32-1.23(2H,m).
MSm/z:400(C₂₆H₂₉N₃O+H).

### (Example 59)

### (-)-2-[3-[N-[(2-Methyl-1,3-thiazol-4-yl)methyl]methylamino] cyclopentyl]-2,2-diphenylacetamide

A mixture of (-)-2-(3-methylaminocyclopentyl)-2,2-diphenylacetamide (0.31 g: 1 mmol), 4-chloromethyl-2-methyl-1,3-thiazole (0.15 g: 1 mmol) , potassium carbonate (0.14 g: 1 mmol) and acetonitrile (10 ml) was stirred for 2 hours under reflux. After concentrating the solvent under reduced pressure, residue was extracted with chloroform and dried over anhydrous potassium carbonate. After concentration under reduced pressure, the residue was purified by silica gel column chromatography [eluant: chloroform/methanol = 20/1] to give the title compound as a colorless amorphous substance (yield: 0.34 g, 81%).
[α]_{D}²⁶:-30.5° (c=1.54,CHCl₃).
¹H-NMR(CDCl₃) δ:7.23-7.41(10H,m),6.87(1H,s),5.46(2H,s),3.47 -3.59(2H,m),3.35-3.42(1H,m),2.83-2.89(1H,m),2.67(3H,s),2.0 5-2.25(1H,m),2.08(3H,s),1.76-1.95(2H,m),1.16-1.43(3H,m).
MSm/z:420(C₂₅H₂₉N₃OS+H).

### (Example 60)

### (-)-2-[3-[N-(5-Methylfurfuryl)methylamino]cyclopentyl]-2,2-diphenylacetamide

(-)-2-(3-methylaminocyclopentyl)-2,2-diphenylacetamide hydrochloride (0.14 g: 0.4 mmol) and 5-methylfurfural (0.04 g: 0.4 mmol) were used for production according to the method of (Example 52) to give the title compound as an oily substance (yield: 0.09 g, 57%).
[α]_{D}²⁸:-27.5° (c=0.73,CHCl₃).
¹H-NMR(CDCl₃) δ:7.16-7.44(10H,m),5.99(1H,d,J=3.05Hz),5.84(1 H,dd,J=3.05,1.22Hz),5.45(2H,brs),3.24-3.50(3H,m),2.62-2.84 (1H,m),2.25(3H,s),2.08(3H,s),1.99-2.14(1H,m),1.62-1.98(2H, m),1.04-1.44(3H,m).
MSm/z:403(C₂₆H₃₀N₂O₂+H).

### (Example 61)

### 2-[3-(1-Methylpiperazin-4-yl)cyclopentyl]-2,2-diphenylacetonitrile

2-(3-Oxocyclopentyl)-2,2-diphenylacetonitrile (0.44 g: 1.6 mmol) and 1-methylpiperazine (0.18 ml: 1.6 mmol) were used for production according to the method of (Example 52) to give the title compound as a white powder (yield: 0.44 g, 77%).
¹H-NMR(CDCl₃) δ:7.08-7.56(10H,m),2.96-3.16(1H,m),2.30(3H,s) ,2.08-2.76(9H,m),1.42-2.01(6H,m).
MSm/z:360(C₂₄H₂₉N₃+H).

### (Example 62)

### 2-[3-(1-Methylpiperazin-4-yl)cyclopentyl]-2,2-diphenylacetamide

The title compound was obtained as a white crystalline powder from 2-[3-(1-methylpiperazin-4-yl)cyclopentyl]-2,2-diphenylacetonitrile (0.42 g: 1.16 mmol) according to the method of (Example 35) (yield: 0.33 g, 75%).
Melting point 154.5-156°C
¹H-NMR(CDCl₃) δ:7.04-7.50(10H,m),5.41(2H,brs),3.24-3.54(1H, m),1.68-2.92(14H,m),0.94-1.47(4H,m).
MSm/z:378(C₂₄H₃₁N₃O+H).

### (Example 63)

### 2-[3-(1-Benzylpiperazin-4-yl)cyclopentyl]-2,2-diphenylacetonitrile

2-(3-Oxocyclopentyl)-2,2-diphenylacetonitrile (1.76 g: 6.4 mmol) and 1-benzylpiperazine (1.1 ml: 6.4 mmol) were used for production according to the method of (Example 52) to give the title compound as a white powder (yield: 2.35 g, 84%).
¹H-NMR(CDCl₃) δ:7.08-7.58(15H,m),3.38-3.56(2H,m),2.96-3.12( 1H,m),2.11-2.88(9H,m),1.44-2.02(6H,m).
MSm/z:436(C₃₀H₃₃N₃+H).

### (Example 64)

### 2-[3-(1-Benzylpiperazin-4-yl)cyclopentyl]-2,2-diphenylacetamide

The title compound was obtained as a colorless amorphous substance from 2-[3-(1-benzylpiperazin-4-yl)cyclopentyl]-2,2-diphenylacetonitrile (2.29 g: 5.26 mmol) according to the method of (Example 35) (yield: 0.75 g, 31%).
¹H-NMR(CDCl₃) δ:7.06-7.50(15H,m),5.42(2H,brs),3.14-3.58(3H, m),2.14-2.82(8H,m),1.64-2.12(3H,m),0.94-1.46(4H,m).
MSm/z:454(C₃₀H₃₅N₃O+H).

### (Example 65)

### 2-[3-(1H-piperazin-4-yl)cyclopentyl]-2,2-diphenylacetamide

A mixture of 2-[3-(1-benzylpiperazin-4-yl)cyclopentyl]-2,2-diphenylacetamide (0.59 g: 1.30 mmol), 10% palladium carbon (0.08 g), 1 N hydrochloric acid (2.6 ml) and ethanol (20 ml) was stirred at room temperature under hydrogen atmosphere for 2 hours. After filtering the insoluble portion, and the filtrate was concentrated, residue was concentrated, residue was extacted with chloroform in the presence of an alkali. After drying over anhydrous sodium sulfate, it was concentrated under reduced pressure to give the title compound as a colorless oil (yield: 0.47 g, quantitative).
¹H-NMR(CDCl₃) δ:7.04-7.52(10H,m),5.44(2H,brs),3.24-3.48(1H, m),2.70-2.92(4H,m),2.52-2.68(1H,m),2.16-2.50(4H,m),1.64-2. 14(4H,m),0.94-1.47(3H,m).

### (Example 66)

### 2-[3-(1-Isopropylpiperazin-4-yl)cyclopentyl]-2,2-diphenylacetamide

2-[3-(1H-Piperazin-4-yl)cyclopentyl]-2,2-diphenylacetamide (0.28 g: 0.77 mmol) and acetone (0.09 g: 1.54 mmol) were used for production according to the method of (Example 52) to give the title compound was obtained as a colorless amorphous substance (yield: 0.22 g, 72%).
¹H-NMR(CDCl₃) δ:7.12-7.52(10H,m),5.47(2H,brs),3.24-3.46(1H, m),2.14-2.82(9H,m),1.68-2.12(3H,m),1.02(6H,d,J=6.71Hz),0.9 2-1.44(4H,m).
MSm/z:406(C₂₆H₃₅N₃O+H).

### (Example 67)

### 2-[3-[1-(4-Methyl-3-pentenyl)piperazin-4-yl]cyclopentyl]-2,2-diphenylacetamide

2-[3-(1H-Piperazin-4-yl)cyclopentyl]-2,2-diphenylacetamide (0.25 g: 0.69 mmol) and 5-bromo-2-methyl-2-pentene (0.11 g: 0.69 mmol) were used for production according to the method of (Example 11) to give the title compound as a colorless amorphous substance (yield: 0.09 g, 28%).
¹H-NMR(CDCl₃) δ:7.08-7.48(10H,m),5.40(2H,brs),4.96-5.14(1H, m),3.24-3.51(1H,m),1.72-2.84(15H,m),1.67(3H,s),1.60(3H,s), 0.92-1.46(4H,m).
MSm/z:446(C₂₉H₃₉N₃O+H).

### (Example 68)

### 2-[3-(Methylamino)cyclopentyl]-2,2-diphenylacetonitrile hydrochloride

A mixture of (+)-2-[3-(N-benzylmethylamino)cyclopentyl]-2,2-diphenylacetonitrile (2.40 g), 1 N hydrochloric acid (10 ml), ethanol (50 ml) and 10% palladium carbon (0.3 g) was stirred at room temperature under a hydrogen atmosphere for one hour. The mixture was filtered ,and then the filtrate was concentrated to give the title compound as colorless crystals (yield: 2.04 g, 99%).
Melting point: 271°C (decomposed)
[α]_{D}²²:+23.5° (c=1.00,CH₃OH).
¹H-NMR(CDCl₃) δ:9.64(2H,brs),7.49-7.24(10H,m),3.55(1H,m),3. 14(1H,m),2.60(3H,brs),2.16(3H,m),1.81(3H,m).
MSm/z:291(C₂₀H₂₂N₂+H).

### (Example 69)

### 2-[3-[N-[[(S)-(N-Tosylpyrrolidin-2-yl)]methyl]methylamino] cyclopentyl]-2,2-diphenylacetonitrile

2-[3-(Methylamino)cyclopentyl]-2,2-diphenylacetonitrile hydrochloride (0.65 g) and (S)-[(N-tosylpyrrolidin-2-yl)methyl] tosylate (0.82 g) were used for production according to the method of (Example 38) to give the title compound as a colorless oil (yield: 0.51 g, 49%).
¹H-NMR(CDCl₃) δ:7.70(2H,d,J=7.9Hz),7.47-7.23(12H,m),3.66(1H ,m),3.38(1H,m),3.05(2H,m),2.85(1H,m),2.60(1H,m),2.42(4H,m), 2.29(3H,s),1.90-1.44(10H,m).

### (Example 70)

### 2-[3-[N-[[(S)-(Pyrrolidin-2- yl)]methyl]methylamino]cyclopentyl]-2,2-diphenylacetonitrile

2-[3-[N-[[(S)-(N-Tosylpyrrolidin-2-yl)]methyl]methylamino] cyclopentyl]-2,2-diphenylacetonitrile (0.20 g) and phenol (0.15 g) were stirred for 2 hours under reflux in 47% hydrobromic acid (5 ml). The cooled reaction mixture was extracted with a mixture of hexane-toluene (1/1), and the aqueous layer was alkalized with an aqueous 10 N sodium hydroxide. The alkaline aqueous solution was extracted with chloroform (3 x 100 ml), dried over anhydrous magnesium sulfate and concentrated to give the title compound (yield: 0.10 g, 71%).
¹H-NMR(CDCl₃) δ:7.45-7.16(10H,m),3.64(1H,m),3.40-3.11(5H,m) ,2.54-2.36(2H,m),2.26(3H,s),2.09-1.45(10H,m).

### (Example 71)

### 2-[3-[N-[[(S)-(N-Methylpyrrolidin-2-yl)]methyl]methylamino] cyclopentyl]-2,2- diphenylacetonitrile

A mixture of 2-[3-[N-[[(S)-(pyrrolidin-2-yl)]methyl] methylamino]cyclopentyl]-2,2-diphenylacetonitrile (0.10 g), formic acid (1.0 ml) and formalin (1.0 ml) was stirred for 5 hours under heated reflux. Water and a sodium hydroxide aqueous solution were added for alkalinity, and then extracted with chloroform (3 x 70 ml). Extract was washed with water, dried over anhydrous magnesium sulfate and concentrated. The oily residue was purified by silica gel column chromatography [eluant: chloroform/methanol = 30/1-10/1] to give the title compound as a colorless oil (yield: 80 mg, 80%).
¹H-NMR(CDCl₃) δ:7.47-6.71(10H,m),3.05(2H,m),2.87(1H,m),2.49 (4H,m),2.32-2.20(3H,m),2.20(3H,s),1.98(1H,m),1.87-1.47(9H,m).

### (Example 72)

### (-)-2-[3-[N-[[(S)-(N-Methylpyrrolidin-2-yl)]methyl]methylamino] cyclopentyl]-2,2-diphenylacetamide

A mixture of 2-[3-[N-[[(S)-(N-methylpyrrolidin-2-yl)]methyl]methylamino]cyclopentyl]-2,2-diphenylacetonitrile (80 mg) and 70% sulfuric acid (3.5 ml) was stirred at 120°C for 2 hours. After cooling, a aqueous sodium hydroxide was added for alkalinity, and extracted with chloroform (4 x 50 ml). Organic solution was washed with water, dried over anhydrous magnesium sulfate and concentrated. The oily residue was purified by silica gel column chromatography [eluant: chloroform/methanol = 10/1] to give the title compound as a colorless oil (yield: 34 mg, 41%).
[α]_{D}³⁰:-60.6° (c=0.42,CHCl₃).
¹H-NMR(CDCl₃) δ:7.39-7.23(10H,m),5.40(2H,brs),3.34(1H,m),3. 22(1H,m),2.86(1H,m),2.45(4H,m),2.27(3H,m),2.10(3H,s),2.01-1.73(6H,m),1.57(1H,m),1.45-1.17(3H,m).
MSm/z:406(C₂₆H₃₅N₃O+H).

### (Example 73)

### 2-[3-[N-[[(R)-(N-Tosylpyrrolidin-2-yl)]methyl]methylamino] cyclopentyl]-2,2-diphenylacetonitrile

2-[3-(Methylamino)cyclopentyl]-2,2-diphenylacetonitrile hydrochloride (0.65 g) and (R)-[(N-tosylpyrrolidin-2-yl)methyl] tosylate (0.82 g) were used for production according to the method of (Example 69) to give the title compound as a colorless oil (yield: 0.49 g, 47%).
¹H-NMR(CDCl₃) δ:7.65(2H,d,J=8.5Hz),7.46(4H,m),7.36-7.24(8H, m),3.62(1H,m),3.41(1H,m),3.04(2H,m),2.82(1H,m),2.57(1H,m), 2.49(4H,m),2.27(3H,s),1.83-1.46(10H,m).

### (Example 74)

### 2-[3-[N-[[(R)-(Pyrrolidin-2-yl)]methyl]methylamino] cyclopentyl]-2,2-diphenylacetonitrile

2-[3-[N-[[(R)-(N-Tosylpyrrolidin-2-yl)]methyl]methylamino] cyclopentyl]-2,2-diphenylacetonitrile (0.49 g) was used for production according to the method of (Example 70) to give the title compound as an oily substance (yield: 0.45 g, quantitative).
¹H-NMR(CDCl₃) δ:7.47-6.94(10H,m),3.60(1H,m),3.09(4H,m),2.36 (6H,m),1.87-1.51(10H,m).

### (Example 75)

### 2-[3-[N-[[(R)-(N-Methylpyrrolidin-2- yl)]methyl]methylamino] cyclopentyl]-2,2-diphenylacetonitrile

2-3-[N-[[(R)-(Pyrrolidin-2-yl)]methyl]methylamino] cyclopentyl]-2,2-diphenylacetonitrile (0.45 g) was used for production according to the method of (Example 71) to give the title compound as a colorless oil (yield: 0.16 g, 44%).
¹H-NMR(CDCl₃) δ:7.47-7.22(10H,m),3.09(2H,m),2.80(1H,m),2.49 (1H,m),2.39(3H,s),2.34(3H,m),2.19(3H,s),2.00(1H,m),1.87-1. 50(9H,m).

### (Example 76)

### (+)-2-[3-[N-[[(R)-(N-Methylpyrrolidin-2-yl)]methyl]methylamino] cyclopentyl]-2,2-diphenylacetamide

2-[3-[N-[[(R)-(N-Methylpyrrolidin-2-yl)]methyl]methylamino] cyclopentyl]-2,2-diphenylacetonitrile (0.16 g) was used for production according to the method of (Example 72) to give the title compound as a colorless oil (yield: 90 mg, 56%).
[α]_{D}³¹:+27.9° (c=1.16,CHCl₃).
¹H-NMR(CDCl₃) δ:7.40-7.26(10H,m),5.41(2H,brs),3.34(1H,m),3. 10(1H,m),2.82(1H,m),2.39(4H,m),2.26(3H,m),2.10(3H,s),2.05-1.69(6H,m),1.53(1H,m),1.35(1H,m),1.21(2H,m).
MSm/z:406(C₂₆H₃₅N₃O+H).

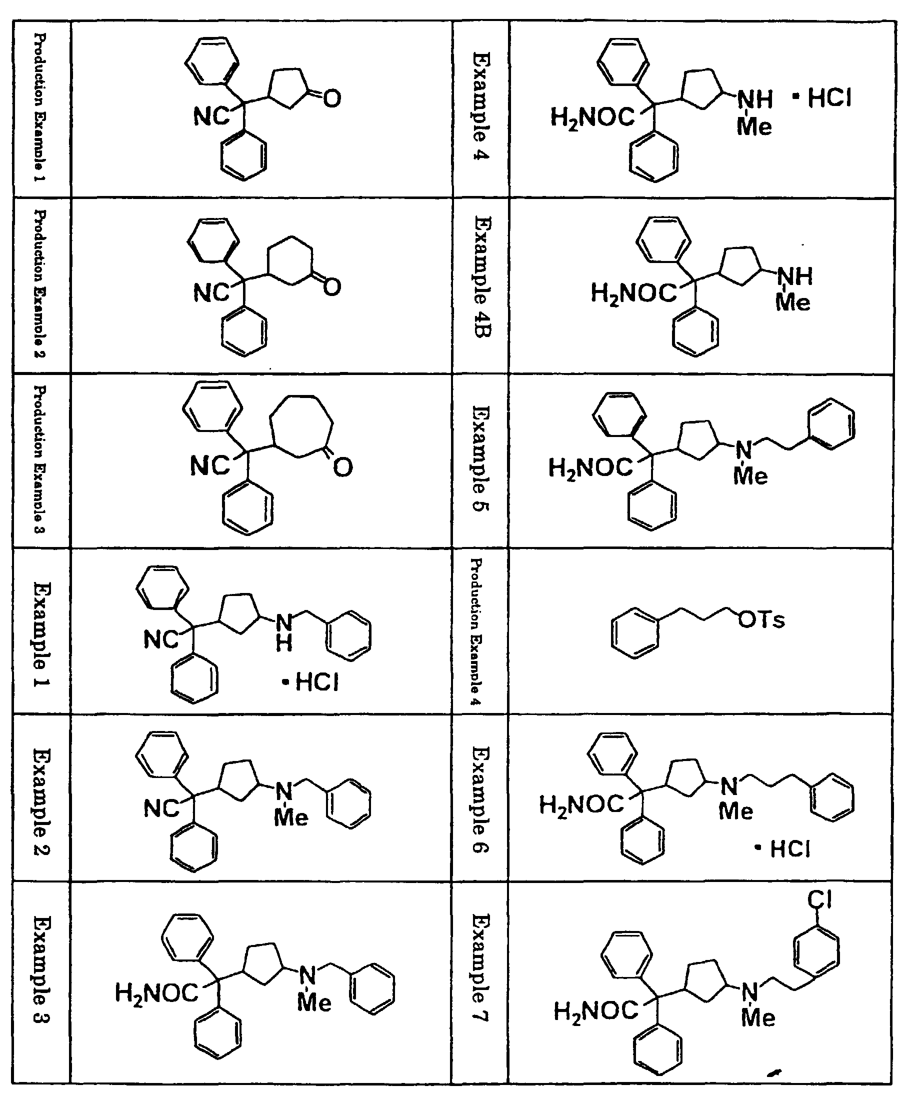

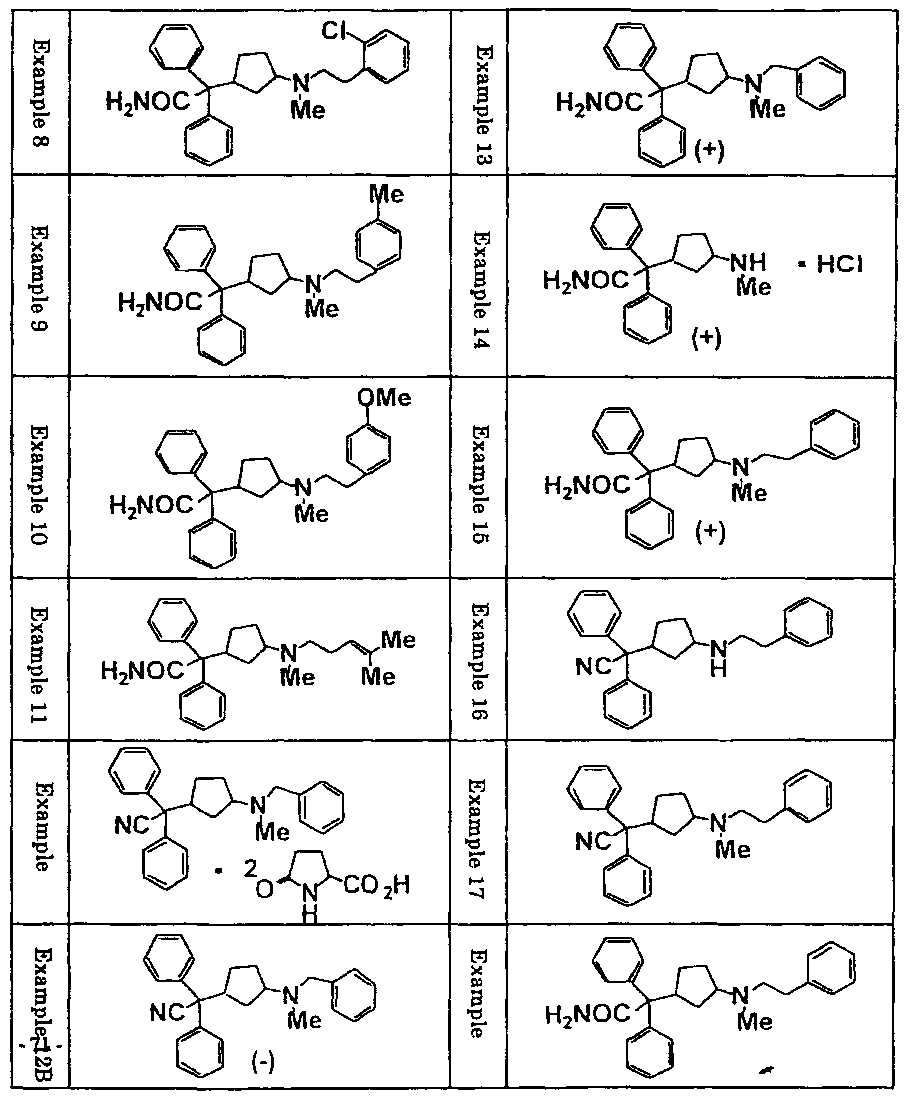

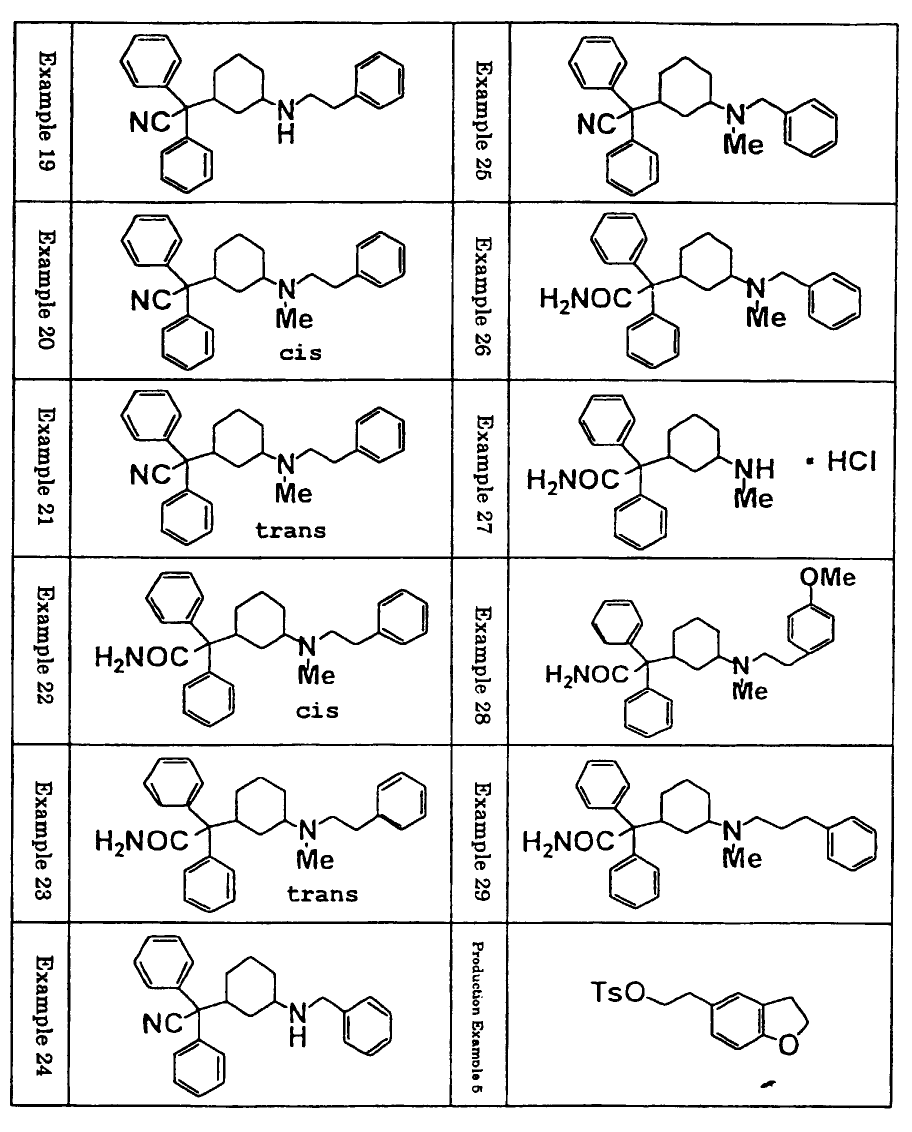

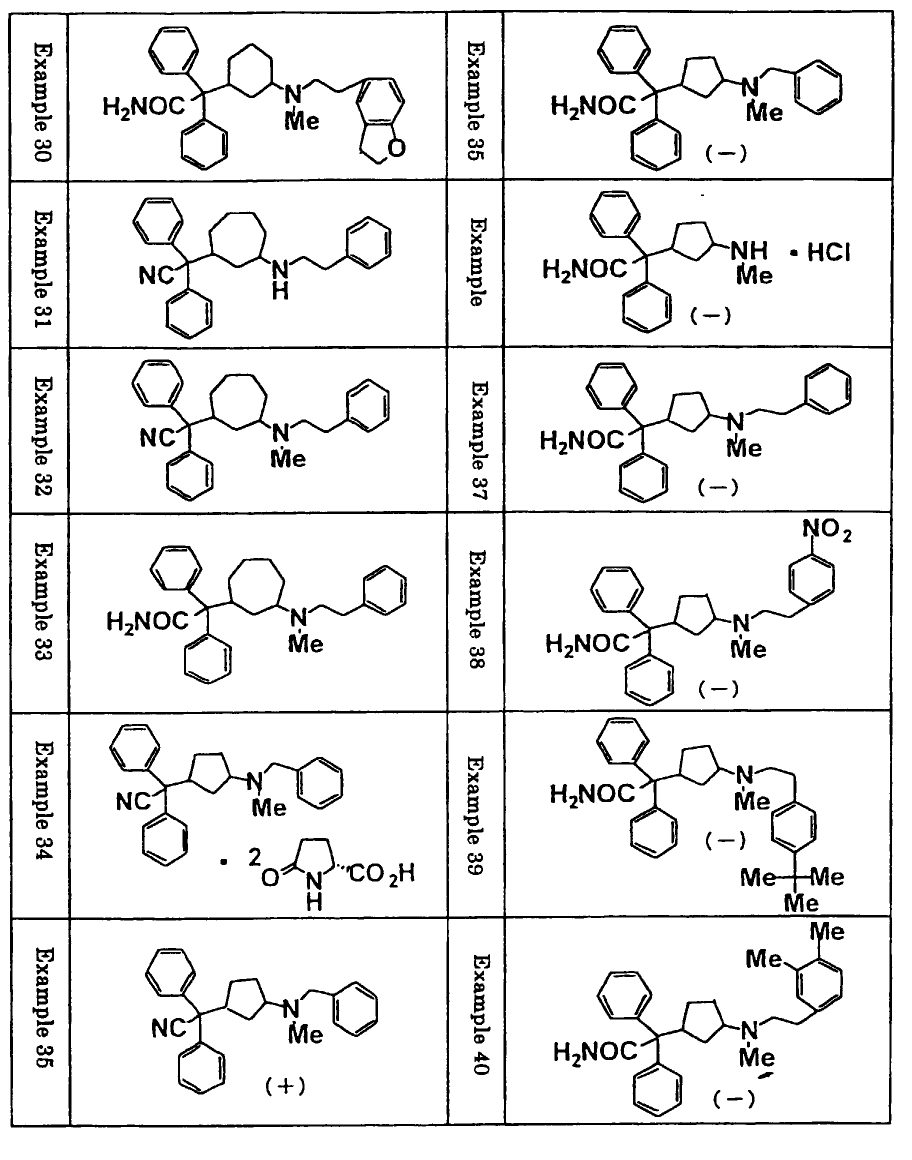

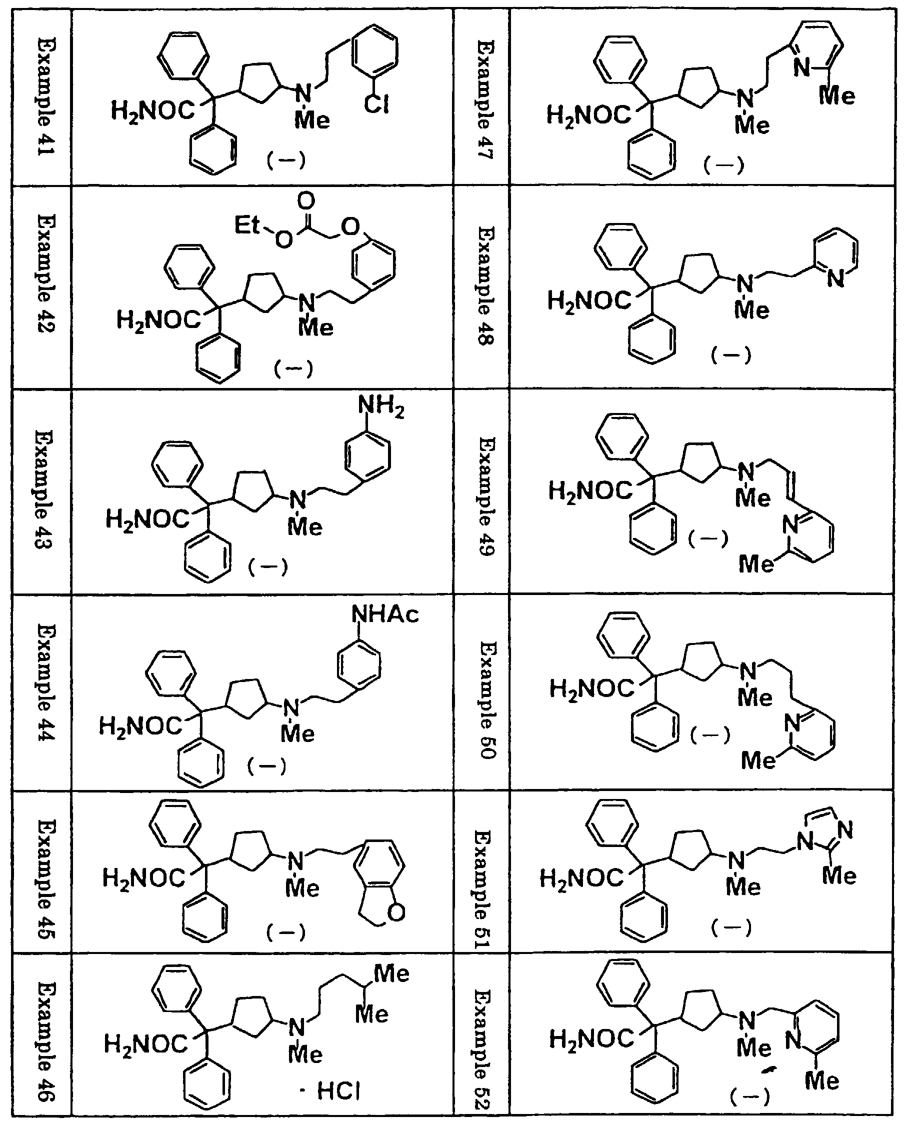

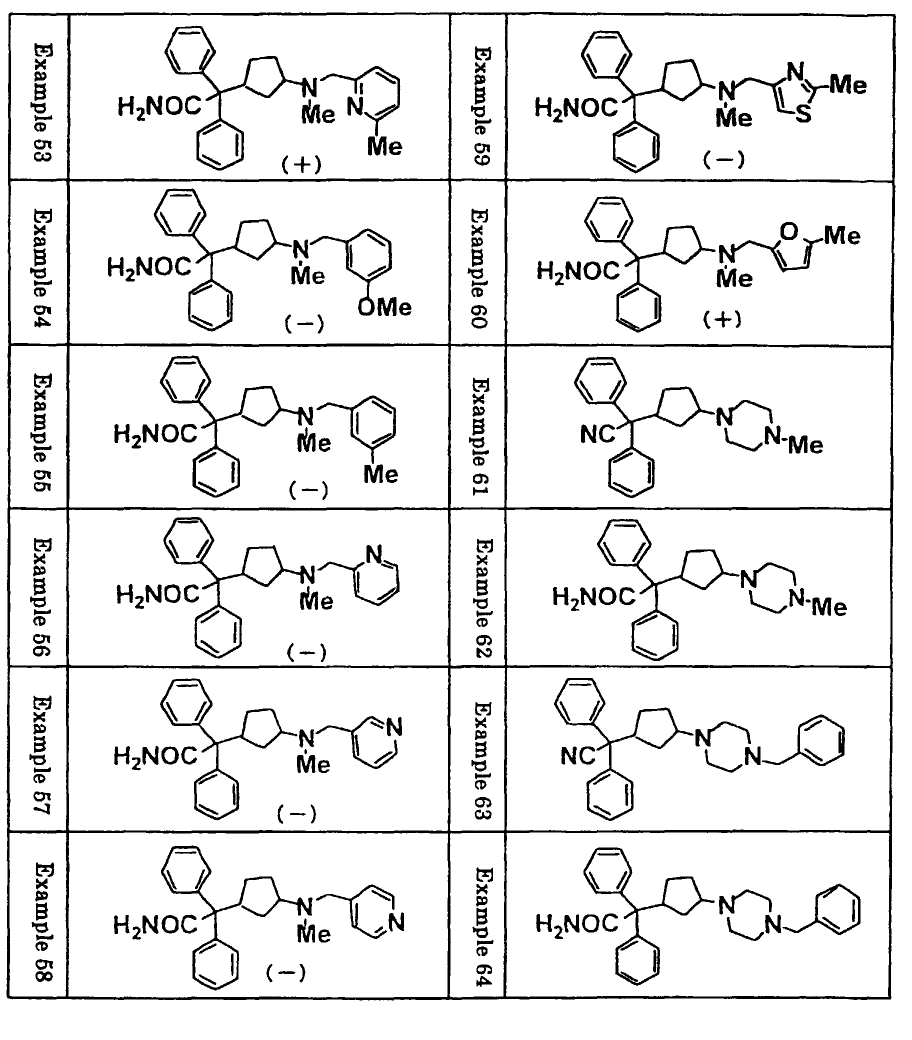

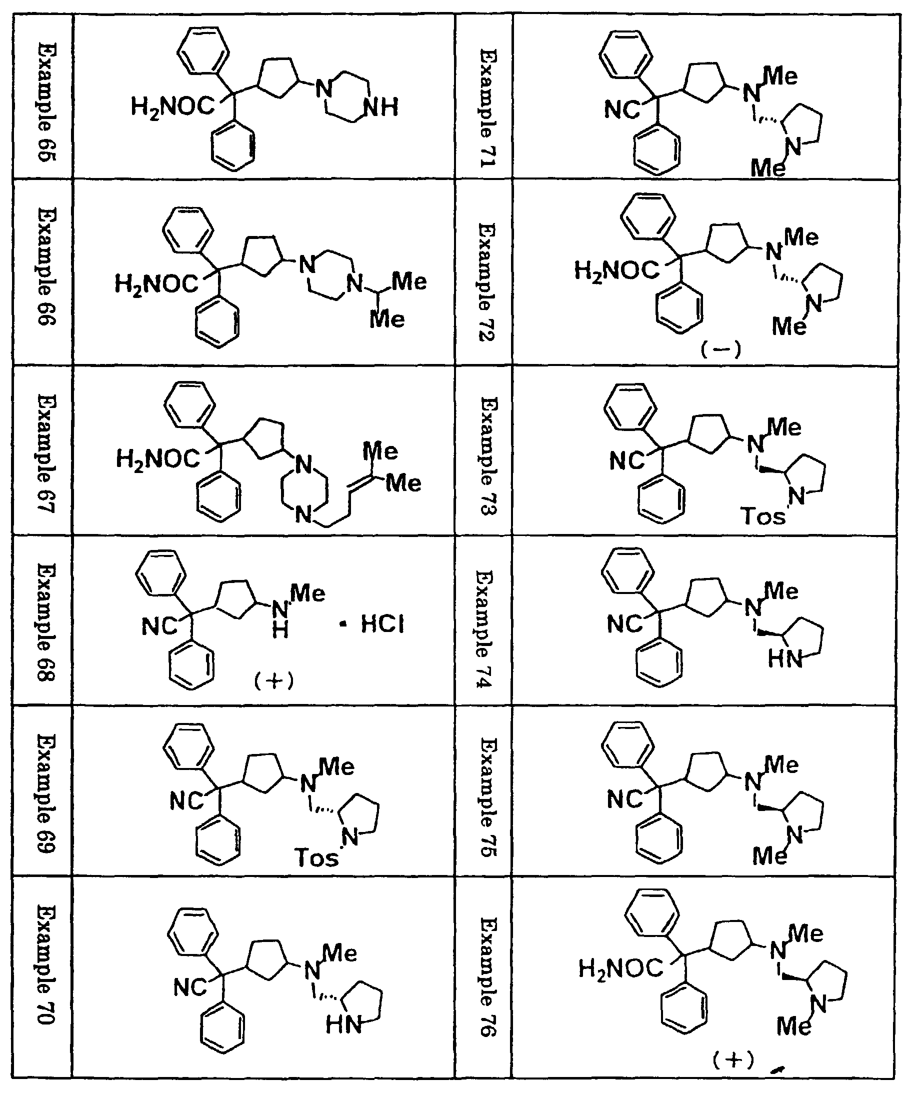

### Effect of the Invention

### 1. Antagonistic action on muscarinic M₃ receptor subtypes in isolated guinea pig longitudinal ileum

Hartley guinea pigs were killed by bleeding after a blow to the head, and then the ileum of each was removed while ablating the mesenterium. The contents of the ileum were thoroughly washed, a glass rod with a diameter of 5-7 mm was inserted into the lumen, the longitudinal muscle alone was cut with a razor along the mesenterium-attached section, a cotton swab was placed at the border between the longitudinal muscle and the circular muscle, and the longitudinal muscle was pulled off as a preparation, taking care to avoid drying of the tissue. The preparation was incubated at 37°C, and after suspending it in a 10 ml organ bath filled with Krebs bicarbonate buffer solution and aerated with 95% O₂-5% CO₂ at a resting tension of 1 g, it was equilibrated for a period of 60 minutes. The carbachol-induced contraction response was recorded at isotonicity using the cumulative method at a common ratio of 3 from 10⁻⁹ M. The preparation was rapidly washed after recording, and equilibrated for a period of 45 minutes until the next contraction response. The point of stabilization of the EC₅₀ of the carbachol-induced contraction response was used as a control. The test compound was applied 15 minutes before carbachol application, and the affinity (pA₂) of the test compound was determined according to the Schild method (Arunlakshana, O. and Schild, H.O.: Brit. J. Pharmacol., 14, 48-58 (1959)). The results are shown in Table 1.

### 2. Antagonistic action on muscarinic M₃ receptor subtypes in isolated guinea pig bladder

Male Hartley guinea pigs were killed by bleeding after a blow to the head, and after abdominal retraction, the visible protruding part of the bladder in the hypogastric region of each was lightly held with forceps while the bladder trigone was cut for removement_and then immersion in a medium. After median dissection, detrusor muscle strips were cut to lengths of 10-15 mm and widths of 3-5 mm. The mucosal tissue was then separated with ophthalmic scissors for use as a preparation. The preparation was incubated at 37°C, and after suspending it in a 10 ml Magnus tank filled with Krebs bicarbonate buffer solution and aerated with 95% O₂-5% CO₂ at a resting tension of 1 g, it was equilibrated for a period of 60 minutes. The carbachol-induced contraction response was recorded at isotonicity using the cumulative method, at a common ratio of 3 from 10⁻⁸ M. The preparation was rapidly washed after recording, and equilibrated for a period of 45 minutes until the next contraction response. The point of stabilization of the EC₅₀ of the carbachol-induced contraction response was used as a control. The test compound was applied 15 minutes before carbachol application. The affinity (pA₂) of the test compound was determined in the same manner as that of the ileum. The results are shown in Table 1.

### 3. Antagonistic action on muscarinic M₃ receptor subtypes in isolated guinea pig trachea

Male Hartley guinea pigs were killed by bleeding after a blow to the head, and the cervical trachea of each was removed while ablating the connective tissue. The esophagus and remaining connective tissue were then amputated and the preparation was prepared by ablation at a spacing of two tracheal cartilages. The preparation was incubated at 37°C, and after suspending it in a 5 ml Magnus tank filled with Krebs bicarbonate buffer solution containing indomethacin (1 µM) and aerated with 95% O₂-5% CO₂ at a resting tension of 1 g, it was equilibrated_for a period of 60 minutes. The carbachol-induced contraction response was recorded at isometricity using the cumulative method, at a common ratio of 3 from 10⁻⁸ M, with 7 minute intervals between each concentration. The preparation was was rapidly washed after recording, and equilibrated for a period of 60 minutes until the next contraction response, with the point of stabilization of the EC₅₀ of the carbachol-induced contraction response used as a control. The test compound was applied 15 minutes before carbachol application. The affinity (pA₂) of the test compound was determined in the same manner as that of the ileum. The results are shown in Table 1.

### 4. Antagonistic action on muscarinic M₂ receptor subtypes in isolated guinea pig left atria

Hartley guinea pigs were killed by bleeding after a blow to the head, and after rapidly removed the heart and lungs of each, ablation was performed in the order of lungs, connective tissue, etc., heart, and the left and right atria were cut apart to prepare the preparation. The preparation_was incubated at 32°C, and then suspended in a 10 ml organ bath filled with Krebs carbonate buffer solution and aerated with 95% O₂-5% CO₂ at a resting tension of 0.5 g. The contraction upon applying a field electric stimulus (4 Hz, 2 msec, 1.5 x threshold voltage) was then recorded. After standing for a stabilization period of 60 minutes, the carbachol-induced inhibition response was recorded at isometricity using the cumulative method, at a common ratio of 3 from 10⁻⁸ M, with 90 second intervals between each concentration. The preparation was rapidly washed after recording, and equilibrated for a period of 45 minutes until the next inhibition response, with the point of stabilization of the EC₅₀ of the carbachol-induced inhibition response used as a control, and the test compound was applied 30 minutes before carbachol application. The affinity (pA₂) of the test compound was determined in the same manner as that of the ileum. The results are shown in Table 1.

As shown in Table 1, the compounds of the invention exhibited selective antagonistic activity on muscarinic M₃ receptor subtypes over muscarinic M₂ receptor subtypes.

**Table 1**

| Muscarinic receptors antagonism activity (in vitro) | | | | |
|---|---|---|---|---|
| Example No. or comparative control compound | pA₂ | | | |
| | Ileum M₃ | Bladder M₃ | Trachea M₃ | Left atrium M₂ |
| 5 | 8.6 | 8.1 | 7.5 | 7.3 |
| 11 | 9.1 | 8.4 | 8.2 | 7.7 |
| 15 | | 7.6 | | 6.7 |
| 37 | 8.8 | 8.2 | | 7.5 |
| 40 | 8.3 | | | |
| 41 | 8.5 | 7.6 | 7.3 | 6.8 |
| 43 | 8.8 | 8.2 | | 7.7 |
| 45 | 8.5 | 8 | | 7 |
| 48 | 9 | 8.2 | 8.7 | 7.1 |
| 52 | 8.4 | 8.5 | 8.6 | 6.2 |
| 55 | 8.5 | 7.8 | 7.4 | 6.4 |
| 59 | 8.8 | 8.4 | 8.9 | 7.3 |
| 60 | 9.3 | 8.5 | 9 | 7.8 |
| Atropine | 8.6 | 8.5 | 8.9 | 8.6 |
| Darifenacin | 8.9 | 8.3 | 7.8 | 7.2 |

### 5. Test in rat cage -restraint stress-induced diarrhea models

Male Wistar rats (8-9 weeks old) that had been starved overnight were orally administered with either the test drug or a solvent (2% ethanol aqueous solution) as a control, and after 30 minutes they were placed under cage restrictions. The condition of the diarrhea was checked for 120 minutes every 30 minutes in each cage, to determine the incidence of diarrhea. The results are shown in Table 2.

**Table 2**

| Effect in rat cage restriction stress-induced diarrhea models (oral administration) | | | | | |
|---|---|---|---|---|---|
| Example No. | Diarrhea incidence(%) | | | | |
| | Control solvent group | 3mg/kg | 10mg/kg | 30mg/kg | Darifenacin 30mg/kg |
| 41 | 75 | | 50 | 13 | 13 |
| 48 | 63 | | 25 | 13 | 35 |
| 48 | 75 | 38 | 25 | | 38 |
| 55 | 88 | | 88 | 13 | 25 |

### 6. Test on rat saliva secretion (oral administration)

Male Wistar rats (8-9 weeks old) that had been starved overnight were orally administered with either the test drug or a solvent (2% ethanol aqueous solution) as a control, and after 30 minutes they were anesthetized with urethane (1.2 g/kg, i.p.) and then the right femoral region of each was dissected, the right femoral vein was exposed and a drug-administering cannula was inserted. After 15 minutes, 100 µg/kg of oxotremorine was intravenously administered through the cannula to induce saliva secretion. Immediately after administration of the oxotremorine, a small cotton ball was inserted into the oral cavity. Salivary secretion was determined for 10 minutes, from the moment of oxotremorine application. The inhibition rate was calculated with respect to the secreted saliva amount in the control group, and the dose of the test compound that inhibited 50% of the secreted saliva amount of the control group was determined as the ID₅₀ value. The results are shown in Tables 3 and 4.

**Table 3**

| Effect on oxotremorine-induced saliva secretion (oral administration) | |
|---|---|
| Example No. or positive control compound | Saliva secretion ID₅₀ mg/kg |
| 41 | 3.7 |
| 48 | 1.7 |
| 55 | 8.2 |
| Darifenacin | 1.1 |

### 7. Test on guinea pig bronchoconstriction (oral administration)

The bronochoconstriction respionse was measured by the Konzett-Rössler method. (Arch. Exp. Path. Pharmak., 195, 71-74, 1940). Male Hartley guinea pigs (270-530 g) were dorsally pinned under urethane (1.8 g/kg, i.p.) anesthesia. The trachea, left common carotid artery and left common jugular vein of each was exposed by cervical median incision, insering a tracheal cannula, sphygmomanometric cannula and drug-administering cannula into the each tissue. Animals were ventilated artificially through a tracheal cannula. Spontaneous respiratory movement was stopped by decamethonium (2 mg/kg, i.v.). The lungs were inflated at a fixed volume of air under a constant pressure (10 cmH₂O). Ventilation outflow was recorded through a bronchospasm transducer as an index of airway resistance. The drug or control solvent (2% ethanol aqueous solution) was orally administered, and after 60 minutes, methacholine (3 µg/kg) was administered through the drug-administering cannula in the left jugular vein, and bronochoconstriction was induced. The Effect of the drug on airway resistance was calculated against 100% as the total obstruction value obtained by closure of the tracheal cannula, and the dose of the test compound that caused 50% inhibition of and bronochoconstriction was recorded as the ID₅₀ value. The results are shown in Table 4.

**Table 4**

| Muscarinic receptors antagonistic action (in vivo) (oral administration) | | |
|---|---|---|
| Example No. or comparison control compound | Bronochoconstricti on ID₅₀ (mg/kg) | Saliva secretion ID₅₀ (mg/kg) |
| 52 | 1.2 | 2.5 |
| Atropine | 1.8 | 0.4 |

### Industrial Applicability

Thus, the compounds of the present invention were demonstrated to have a powerful suppressing effect on diarrhea and a powerful inhibiting effect on bronochoconstriction. Their inducement of mouth dryness was also shown to be weaker than that of Darifenacin or atropine used as the control compounds. The compounds of the invention are therefore useful as agents with antagonistic action on muscarinic M₃ receptor subtypes with milder side effects, and can therefore be used as preventive or therapeutic agents for diseases related to muscarinic M₃ receptor subtypes, and particularly digestive tract diseases such as irritable bowel syndrome, spastic colitis and diverticulitis; central nervous system conditions such as drug-induced nausea, vomiting, motin sickness_and Meniere's disease; respiratory diseases such as chronic obstructive pulmonary disease, chronic bronchitis, asthma, pulmonary fibrosis and rhinitis; and urologic diseases such as urinary incontinence and frequent urination, etc.

## Claims

1. An aminocycloalkane compound represented by the following general formula: 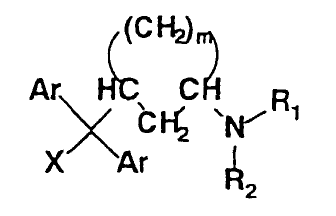 wherein Ar represents a phenyl group or thienyl group with an optional substituent on the ring, X represents a cyano group or carbamoyl group, R₁ and R₂ are each independent, R₁ representing a hydrogen atom or lower alkyl group and R₂ representing a hydrogen atom, lower alkyl group, an -A-B group (where A represents a lower alkylene group that is optionally branched and B represents a phenyl group, pyridyl group, thiazole group, imidazole group, furyl group or pyrrolidinyl group with an optional substituent on the ring) or an alkenyl group, or R₁ and R₂ together with the nitrogen atom to which they are bonded represent 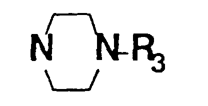 (where R₃ represents a hydrogen atom, a lower alkyl group that is optionally substituted with a phenyl group, or an alkenyl group), and m represents 2, 3 or 4, or a pharmacologically acceptable salt thereof.

2. A compound or pharmacologically acceptable salt thereof according to Claim 1, wherein Ar is a phenyl group.

3. A compound or pharmacologically acceptable salt thereof according to Claim 1 or 2, wherein A is an alkylene group of 1 to 6 carbon atoms.

4. A compound or pharmacologically acceptable salt thereof according to Claim 3, wherein A is a methylene group, ethylene group or trimethylene group.

5. A compound or pharmacologically acceptable salt thereof according to any of Claims 1 to 4, wherein the substituent on the ring of B is a monovalent or divalent substituent.

6. A compound or pharmacologically acceptable salt thereof according to Claim 5, wherein the substituent on the ring of B is a halogen atom, nitro group, amino group, lower alkyl group, lower alkoxy group, lower acylamino group, cyano group, lower alkoxycarbonylalkoxy group or ethyleneoxy group.

7. A compound or pharmacologically acceptable salt thereof according to any of Claims 1 to 6, wherein the general formula represents 2-[3-(N-phenethylmethylamino)cyclopentyl]-2,2-diphenylacetamide, 2-[3-[N-[2-(p-tolyl)ethyl]methylamino] cyclopentyl]-2,2-diphenylacetamide, 2-[3-[N-[2-(4-methoxyphenyl)ethyl]methylamino]cyclopentyl]-2,2- diphenylacetamide, 2-[3-[N-(4-methylpenten-3-yl)methylamino]cyclopentyl]-2,2-diphenylacetamide, 2-[3-[N-[2-(3-chlorophenyl)ethyl]methylamino]cyclopentyl]-2,2-diphenylacetamide, 2-[3-[N-(4-methylpentyl)methylamino] cyclopentyl]-2,2-diphenylacetamide, 2-[3-[N-[2-(pyridin-2-yl)ethyl]methylamino]cyclopentyl]-2,2-diphenylacetamide, 2-[3-[N-[(6-methylpyridin-2-yl)methyl]methylamino]cyclopentyl]-2,2-diphenylacetamide, 2-[3-[N-(3-methylbenzyl)methylamino] cyclopentyl]-2,2-diphenylacetamide, 2-[3-[N-[(2-methyl-1,3-thiazol-4-yl)methyl]methylamino]cyclopentyl]-2,2-diphenylacetamide or 2-[3-[N-(5-methylfurfuryl)methylamino] cyclopentyl]-2,2-diphenylacetamide.

8. A compound or pharmacologically acceptable salt thereof according to any of Claims 1 to 7, wherein the general formula represents (-)-2-[3-[N-[2-(3-chlorophenyl)ethyl]methylamino] cyclopentyl]-2,2-diphenylacetamide, (-)-2-[3-[N-[2-(pyridin-2-yl)ethyl]methylamino]cyclopentyl]-2,2-diphenylacetamide, (-)-2-[3-[N-[(6-methylpyridin-2-yl)methyl]methylamino]cyclopentyl]-2,2-diphenylacetamide or (-)-2-[3-[N-(3-methylbenzyl) methylamino]cyclopentyl]-2,2-diphenylacetamide.

9. A pharmaceutical composition comprising as an effective component thereof an aminocycloalkane compound represented by the following general formula: 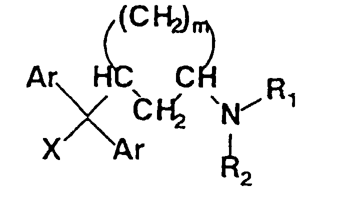 wherein Ar represents a phenyl group or thienyl group with an optional substituent on the ring, X represents a cyano group or carbamoyl group, R₁ and R₂ are each independent, R₁ representing a hydrogen atom or lower alkyl group and R₂ representing a hydrogen atom, lower alkyl group, an -A-B group (where A represents a lower alkylene group that is optionally branched and B represents a phenyl group, pyridyl group, thiazole group, imidazole group, furyl group or pyrrolidinyl group with an optional substituent on the ring) or an alkenyl group, or R₁ and R₂ together with the nitrogen atom to which they are bonded represent 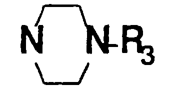 (where R₃ represents a hydrogen atom, a lower alkyl group that is optionally substituted with a phenyl group, or an alkenyl group), and m represents 2, 3 or 4, or a pharmacologically acceptable salt thereof.

10. A pharmaceutical composition according to Claim 9, wherein Ar is a phenyl group.

11. A pharmaceutical composition according to Claim 9 or 10, wherein A is an alkylene group of 1 to 6 carbon atoms.

12. A pharmaceutical composition according to Claim 11, wherein A is a methylene group, ethylene group or trimethylene group.

13. A pharmaceutical composition according to any of Claims 9 to 12, wherein the substituent on the ring of B is a monovalent or divalent substituent.

14. A pharmaceutical composition according to Claim 13, wherein the substituent on the ring of B is a halogen atom, nitro group, amino group, lower alkyl group, lower alkoxy group, lower acylamino group, cyano group, lower alkoxycarbonylalkoxy group or ethyleneoxy group.

15. A pharmaceutical composition according to any of Claims 9 to 14, wherein the general formula represents 2-[3-(N-phenethylmethylamino)cyclopentyl]-2,2-diphenylacetamide, 2-[3-[N-[2-(p-tolyl)ethyl]methylamino] cyclopentyl]-2,2-diphenylacetamide, 2-[3-[N-[2-(4-methoxyphenyl)ethyl] methylamino]cyclopentyl]-2,2-diphenylacetamide, 2-[3-[N-(4-methylpenten-3-yl)methylamino]cyclopentyl]-2,2- diphenylacetamide, 2-[3-[N-[2-(3-chlorophenyl)ethyl] methylamino]cyclopentyl]-2,2-diphenylacetamide, 2-[3-[N-(4-methylpentyl)methylamino] cyclopentyl]-2,2-diphenylacetamide, 2-[3-[N-[2-(pyridin-2-yl)ethyl]methylamino]cyclopentyl]-2,2-diphenylacetamide, 2-[3-[N-[(6-methylpyridin-2-yl)methyl]methylamino]cyclopentyl]-2,2-diphenylacetamide, 2-[3-[N-(3-methylbenzyl)methylamino] cyclopentyl]-2,2-diphenylacetamide, 2-[3-[N-[(2-methyl-1,3-thiazol-4-yl)methyl]methylamino]cyclopentyl]-2,2-diphenylacetamide or 2-[3-[N-(5-methylfurfuryl)methylamino] cyclopentyl]-2,2-diphenylacetamide.

16. A pharmaceutical composition according to any of Claims 9 to 15, wherein the general formula represents (-)-2-[3-[N-[2-(3-chlorophenyl)ethyl]methylamino] cyclopentyl]-2,2-diphenylacetamide, (-)-2-[3-[N-[2-(pyridin-2-yl)ethyl]methylamino]cyclopentyl]-2,2-diphenylacetamide, (-)-2-[3-[N-[(6-methylpyridin-2-yl)methyl]methylamino]cyclopentyl]-2,2-diphenylacetamide or (-)-2-[3-[N-(3-methylbenzyl) methylamino]cyclopentyl]-2,2-diphenylacetamide.

17. A pharmaceutical composition according to any of Claims 9 to 16, which is a preventive or therapeutic agent for a disease related to muscarinic M₃ receptor subtypes.

18. A pharmaceutical composition according to any of Claims 9 to 17, which is a preventive or therapeutic agent for a digestive tract disease, respiratory disease, urologic disease or central nervous system condition.

19. A pharmaceutical composition according to Claim 18, wherein the digestive tract disease is irritable bowel syndrome, spastic colitis or diverticulitis.

20. A pharmaceutical composition according to Claim 18, wherein the respiratory disease is chronic obstructive pulmonary disease, asthma, pulmonary fibrosis or rhinitis.

21. A pharmaceutical composition according to Claim 18, wherein the urologic disease is urinary incontinence or frequent urination.

22. A pharmaceutical composition according to Claim 18, wherein the central nervous system condition is drug-induced nausea, vomiting, motin sickness or Meniere's disease.

23. An agent with antagonistic action on muscarinic M₃ receptor subtypes comprising as an effective component thereof an aminocycloalkane compound represented by the following general formula: 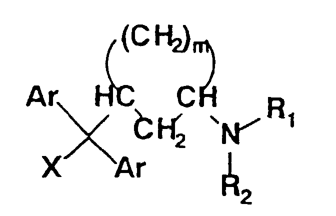 wherein Ar represents a phenyl group or thienyl group with an optional substituent on the ring, X represents a cyano group or carbamoyl group, R₁ and R₂ are each independent, R₁ representing a hydrogen atom or lower alkyl group and R₂ representing a hydrogen atom, lower alkyl group, an -A-B group (where A represents a lower alkylene group that is optionally branched and B represents a phenyl group, pyridyl group, thiazole group, imidazole group, furyl group or pyrrolidinyl group with an optional substituent on the ring) or an alkenyl group, or R₁ and R₂ together with the nitrogen atom to which they are bonded represent 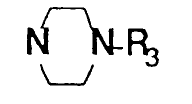 (where R₃ represents a hydrogen atom, a lower alkyl group that is optionally substituted with a phenyl group, or an alkenyl group), and m represents 2, 3 or 4, or a pharmacologically acceptable salt thereof.
